# EUROPEAN PATENT SPECIFICATION

(11) **EP 4 419 513 B1**
(45) Date of publication and mention of the grant of the patent: **27.08.2025**
(21) Application number: 22808714.4
(22) Date of filing: 21.10.2022
(51) Int. Cl.: C07D 309/32, A61P 35/00, A61K 31/351

(54) **ANTITUMORAL COMPOUNDS**
ANTITUMORVERBINDUNGEN
COMPOSÉS ANTITUMORAUX

(30) Priority: 22.10.2021 EP 21382950
(43) Date of publication of application: 28.08.2024
(73) Proprietor: Pharma Mar S.A., 28770 Madrid (ES)
(72) Inventor: MATEO URBANO, María Cristina, 28770 Colmenar Viejo, Madrid (ES); RODRÍGUEZ VICENTE, Alberto, 28770 Colmenar Viejo, Madrid (ES); HERNANDO GARCÍA, Juan, 28770 Colmenar Viejo, Madrid (ES); FRANCESCH SOLLOSO, Andrés Manuel, 28770 Colmenar Viejo, Madrid (ES); AVILÉS MARÍN, Pablo Manuel, 28770 Colmenar Viejo, Madrid (ES); GUILLÉN NAVARRO, María José, 28770 Colmenar Viejo, Madrid (ES); CUEVAS MARCHANTE, María del Carmen, 28770 Colmenar Viejo, Madrid (ES)
(74) Representative: ABG Intellectual Property Law, S.L.
(86) International application number: PCT/EP2022/079359
(87) International publication number: WO 2023/067132

(56) References cited:
- WO-A1-2007/144423
- MARTÍNEZ-DÍEZ MARTA ET AL: "PM060184, a new tubulin binding agent with potent antitumor activity including P-glycoprotein over-expressing tumors", BIOCHEMICAL PHARMACOLOGY, ELSEVIER, US, vol. 88, no. 3, 31 January 2014 (2014-01-31), pages 291 - 302, XP028631446, ISSN: 0006-2952, DOI: 10.1016/J.BCP.2014.01.026

## Description

### FIELD OF THE INVENTION

The present invention relates to novel microtubule inhibitors, pharmaceutical compositions containing them, methods for their manufacture and their use as antitumoral agents.

### BACKGROUND OF THE INVENTION

A novel class of polyketides were identified in the Madagascan sponge *Lithoplocamia lithistoides* including compound PM060184 (PM184) of formula:

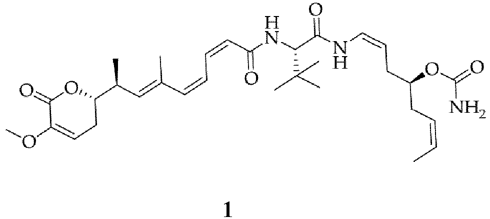

WO 2007/144423 discloses PM184 (compound 1) and other natural or synthetic polyketides. PM184 is a microtubule inhibitor that targets a protein called tubulin via a novel mechanism. It blocks cancer growth by impairing cell division of tumor cells through mitosis inhibition. PM184 is currently undergoing a Phase II trial for hormone-receptor positive, HER2-negative locally advanced and/or metastatic breast cancer and in studies in various solid tumors and in other Phase II solid tumor trials.

PM184 suppresses microtubule shortening and growing to a similar extent, interfering with the dynamic instability of microtubules and affecting cells, both in interphase and mitosis. It is believed that these effects are derived from a new form of interaction with the tubulin dimer, sharing a common tubulin-binding site with rhizoxin and concomitantly interfering with the binding of vinblastine. The unparalleled binding mode on tubulin was preliminarily suggested in PM060184-resistant mutants of Aspergillus nidulans, which involves the interaction with a new locus in β-tubulin defined by the position Asn100. This was later confirmed by X-ray crystallography, corroborating the discovery of the maytansine binding site as a new pharmacophore. In addition to the effects on the microtubule network, the antitumor activity of PM060184 may also depend on relevant anti-angiogenic properties, namely the inhibition of migration and invasion described in human umbilical vein endothelial (HUVEC) cells.

Despite the positive results obtained in clinical applications in chemotherapy, the search in the field of oncology is still open to the identification of new compounds with optimal features of activity, selectivity toward the tumour, with a reduced systemic toxicity and/or improved pharmacokinetic properties.

### SUMMARY OF THE INVENTION

In a first aspect of the present invention there is provided a compound of formula I: 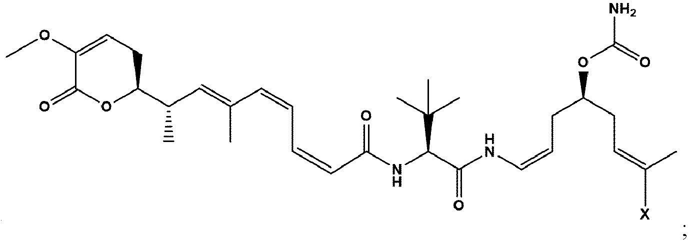 wherein X = F or Me.

In a further aspect of the present invention there is provided a compound of formula II thereof:

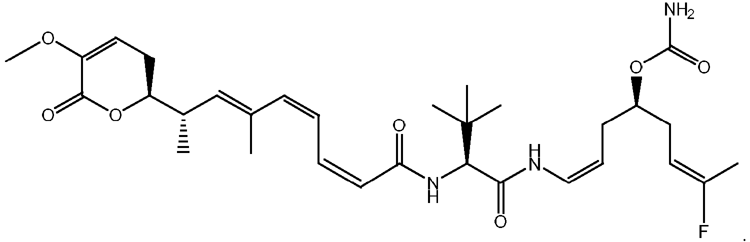

In a further aspect of the present invention there is provided a compound of formula III thereof:

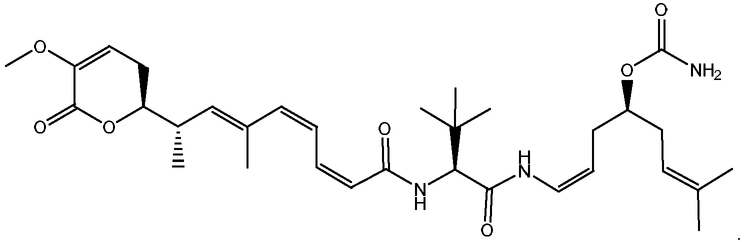

In a further aspect of the present invention, there is provided a pharmaceutical composition comprising a compound according to the present invention and a pharmaceutically acceptable carrier.

In a yet further aspect of the present invention, there is provided a dosage form comprising a pharmaceutical composition according to the present invention.

In a yet further aspect of the present invention, there is provided a compound, pharmaceutical composition or dosage form according to the present invention for use as a medicament.

In a yet further aspect of the present invention, there is provided a compound, pharmaceutical composition or dosage form according to the present invention for use in the treatment of cancer. The cancer may be a solid tumor. The cancer may be selected from lung cancer, including non-small cell lung cancer, colorectal cancer, breast cancer, pancreatic cancer and gastric cancer.

In a yet further aspect of the present invention, there is provided the use of a compound, pharmaceutical composition or dosage form according to the present invention for the manufacture of a medicament for the treatment of cancer. The cancer may be a solid tumor. The cancer may be selected from lung cancer, non-small cell lung cancer, colorectal cancer, breast cancer, pancreatic cancer and gastric cancer.

In a yet further aspect of the present invention, there is provided an in-vitro method of inhibiting cancer cell growth, comprising contacting cancer cells with a compound according to the present invention, or a pharmaceutical composition according to the present invention, or a dosage form according to the present invention; optionally wherein the cancer cell is a solid tumor; including a cancer selected from lung cancer, non-small cell lung cancer, colorectal cancer, breast cancer, pancreatic cancer and gastric cancer.

In a yet further aspect of the present invention, there is provided a kit comprising a therapeutically effective amount of a compound according to the present invention and a pharmaceutically acceptable carrier, or a pharmaceutical composition according to the present inention, or a dosage form according to the present invention. The kit is for use in the treatment of cancer. The cancer may be a solid tumor. The cancer may be selected from lung cancer, non-small cell lung cancer, colorectal cancer, breast cancer, pancreatic cancer and gastric cancer.

In a yet further aspect of the present invention, there is provided a process for the manufacture of a compound of formula **I,** or a pharmaceutically acceptable salt thereof, the process comprising reacting a compound of formula V with a suitable reagent for the synthesis of carbamates to give a compound of formula I 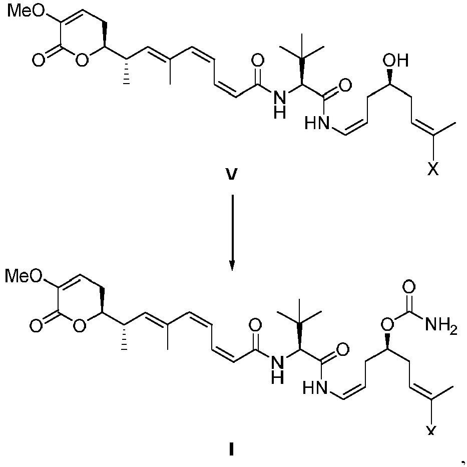 wherein X = F or Me.
In a yet further aspect of the present invention, there are provided intermediate compounds of formula **V, VII, IX** or **XI** useful for the manufacture of a compound according to the present invention.

### BRIEF DESCRIPTION OF THE FIGURES

**Figure 1****.** Tumor growth (median) curve for mice (N = 10) bearing H460 xenografts and treated with PM050489.
**Figure 2****.** Tumor growth (median) curve for mice (N = 10) bearing H460 xenografts and treated with PM060184, **2** and **3;**
**Figure 3****.** Kaplan-Meier survival curve for mice (N = 10) bearing H460 xenografts and treated with PM060184, **2** and **3;**
**Figure 4****.** Tumor growth (median) curve for mice (N = 8) bearing HGC-27 xenografts and treated with PM060184, **2** and **3;**
**Figure 5****.** Kaplan-Meier survival curve for mice (N = 8) bearing HGC-27 xenografts and treated with PM060184, **2** and **3;**
**Figure 6****.** Tumor growth (median) curve for mice (N = 10) bearing HCT-116 xenografts and treated with PM060184, **2** and **3;**
**Figure 7****.** Kaplan-Meier survival curve for mice (N = 10) bearing HCT-116 xenografts and treated with PM060184, **2** and **3.**

### DETAILED DESCRIPTION OF PREFERRED EMBODIMENTS

The following apply to all aspects of the present invention:
The compounds of the invention may be in crystalline or amorphous form either as free compounds or as solvates (e.g. hydrates) and it is intended that all forms are within the scope of the present invention. Methods of solvation are generally known within the art.

In addition, compounds referred to herein may exist in isotopically-labelled forms. All isotopically labelled forms of the compounds referred to herein, and mixtures thereof, are considered within the scope of the present invention.

Protected forms of the compounds disclosed herein are considered within the scope of the present disclosure. Suitable protecting groups are well known for the skilled person in the art. A general review of protecting groups in organic chemistry is provided by Wuts, PGM and Greene TW in Protecting Groups in Organic Synthesis, 4th Ed. Wiley-Interscience, and by Kocienski PJ in Protecting Groups, 3rd Ed. Georg Thieme Verlag. These references provide sections on protecting groups for OH and amino groups.

Within the scope of the present invention an OH protecting group is defined to be the *O-*bonded moiety resulting from the protection of the OH through the formation of a suitable protected OH group. Examples of such protected OH groups include ethers, silyl ethers, esters, sulfonates, sulfenates and sulfinates, carbonates, and carbamates. In the case of ethers the protecting group for the OH can be selected from methyl, methoxymethyl, methylthiomethyl, (phenyldimethylsilyl)methoxymethyl, benzyloxymethyl, *p*-methoxybenzyloxymethyl, [(3,4-dimethoxybenzyl)oxy]methyl, *p*-nitrobenzyloxymethyl, *o-*nitrobenzyloxymethyl, [(*R*)-1-(2-nitrophenyl)ethoxy]methyl, (4-methoxyphenoxy)methyl, guaiacolmethyl, [(*p-*phenylphenyl)oxy]methyl, *t*-butoxymethyl, 4-pentenyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthoxymethyl, *O*-bis(2-acetoxy-ethoxy)methyl, tetrahydropyranyl, fluorous tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxide, 1-[(2-chloro-4-methyl)-phenyl]-4-methoxypiperidin-4-yl, 1-(2-fluorophenyl)-4-methoxypiperidin-4-yl, 1-(4-chlorophenyl)-4-methoxypiperidin-4-yl, 1,4-dioxan-2-yl, tetrahydrofuranyl, tetrahydrothiofuranyl, 2,3,3*a*,4,5,6,7,7*a*-octahydro-7,8,8-trimethyl-4,7-methanobenzofuran-2-yl, 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-dianisyl-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-(phenylselenyl)ethyl, *t*-butyl, cyclohexyl, 1-methyl-1'-cyclopropylmethyl, allyl, prenyl, cinnamyl, 2-phenallyl, propargyl, *p*-chlorophenyl, *p-*methoxyphenyl, *p*-nitrophenyl, 2,4-dinitrophenyl, 2,3,5,6-tetrafluoro-4-(trifluoromethyl)phenyl, benzyl, *p*-methoxybenzyl, 3,4-dimethoxybenzyl, 2,6-dimethoxybenzyl, *o*-nitrobenzyl, *p-*nitrobenzyl, pentadienylnitrobenzyl, pentadienylnitropiperonyl, halobenzyl, 2,6-dichlorobenzyl, 2,4-dichlorobenzyl, 2,6-difluorobenzyl, *p*-cyanobenzyl, fluorous benzyl, 4-fluorousalkoxybenzyl, trimethylsilylxylyl, *p*-phenylbenzyl, 2-phenyl-2-propyl, *p-*acylaminobenzyl, *p*-azidobenzyl, 4-azido-3-chlorobenzyl, 2-trifluoromethylbenzyl, 4-trifluoromethylbenzyl, *p*-(methylsulfinyl)benzyl, *p*-siletanylbenzyl, 4-acetoxybenzyl, 4-(2-trimethylsilyl)ethoxymethoxybenzyl, 2-naphthylmethyl, 2-picolyl, 4-picolyl, 3-methyl-2-picolyl *N*-oxide, 2-quinolinylmethyl, 6-methoxy-2-(4-methylphenyl)-4-quinolinemethyl, 1-pyrenylmethyl, diphenylmethyl, 4-methoxydiphenylmethyl, 4-phenyldiphenylmethyl, *p,p'-*dinitrobenzhydryl, 5-dibenzosuberyl, triphenylmethyl, tris(4-*t*-butylphenyl)methyl, α-naphthyldiphenylmethyl, *p*-methoxyphenyldiphenylmethyl, di(*p*-methoxyphenyl)phenyl-methyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4''-tris(benzoyloxyphenyl)methyl, 4,4'-dimethoxy-3"-[*N*-(imidazolylmethyl)]trityl, 4,4'-dimethoxy-3"-[*N*-(imidazolylethyl)carbamoyl]trityl, bis(4-methoxyphenyl)-1'-pyrenylmethyl, 4-(17-tetrabenzo[*a*,*c*,*g*,*i*]fluorenylmethyl)-4,4"-dimethoxytrityl, 9-anthryl, 9-(9-phenyl)xanthenyl, 9-phenylthioxanthyl, 9-(9-phenyl-10-oxo)anthryl, 1,3-benzodithiolan-2-yl, 4,5-bis(ethoxycarbonyl)-[1,3]-dioxolan-2-yl, benzisothiazolyl S,S-dioxide. In the case of silyl ethers the protecting group for the OH can be selected from trimethylsilyl, triethylsilyl, triisopropylsilyl, dimethylisopropylsilyl, diethylisopropylsilyl, dimethylhexylsilyl, 2-norbomyldimethylsilyl, *t*-butyldimethylsilyl, *t*-butyldiphenylsilyl, tribenzylsilyl, tri-*p*-xylylsilyl, triphenylsilyl, diphenylmethylsilyl, di-*t*-butylmethylsilyl, bis(*t*-butyl)-1-pyrenylmethoxysilyl, tris(trimethylsilyl)silyl, (2-hydroxystyryl)dimethylsilyl, (2-hydroxystyryl)diisopropylsilyl, *t-*butylmethoxyphenylsilyl, *t*-butoxydiphenylsilyl, 1,1,3,3-tetraisopropyl-3-[2-(triphenylmethoxy) ethoxy]disiloxane-1-yl, and fluorous silyl. In the case of esters the protecting group for the OH together with the oxygen atom of the unprotected OH to which it is attached form an ester that can be selected from formate, benzoylformate, acetate, chloroacetate, dichloroacetate, trichloroacetate, trichloroacetamidate, trifluoroacetate, methoxyacetate, triphenylmethoxyacetate, phenoxyacetate, *p*-chlorophenoxyacetate, phenylacetate, diphenylacetate, 3-phenylpropionate, bisfluorous chain type propanoyl, 4-pentenoate, 4-oxopentanoate, 4,4-(ethylenedithio)pentanoate, 5[3-bis(4-methoxyphenyl)hydro-xymethylphenoxy]levulinate, pivaloate, 1-adamantoate, crotonate, 4-methoxycrotonate, benzoate, *p*-phenylbenzoate, 2,4,6-trimethylbenzoate, 4-bromobenzoate, 2,5-difluorobenzoate,*p-*nitrobenzoate, picolinate, nicotinate, 2-(azidomethyl)benzoate, 4-azido-butyrate, (2-azidomethyl)phenylacetate, 2-{[(tritylthio)oxy]methyl}benzoate, 2-{[(4-methoxytritylthio)oxy]methyl}benzoate, 2-{[methyl(tritylthio)amino]methyl}benzoate, 2-{{[(4-methoxytrityl)thio]methylamino}methyl}benzoate, 2-(allyloxy)phenylacetate, 2-(prenyloxymethyl)benzoate, 6-(levulinyloxymethyl)-3-methoxy-2-nitrobenzoate, 6-(levulinyloxymethyl)-3-methoxy-4-nitrobenzoate, 4-benzyloxybutyrate, 4-trialkylsilyloxy-butyrate, 4-acetoxy-2,2-dimethylbutyrate, 2,2-dimethyl-4-pentenoate, 2-iodobenzoate, 4-nitro-4-methylpentanoate, *o*-(dibromomethyl)benzoate, 2-formylbenzenesulfonate, 4-(methylthio-methoxy)butyrate, 2-(methylthiomethoxymethyl)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenyl-acetate, isobutyrate, monosuccinoate, (*E*)-2-methyl-2-butenoate, *o*-(methoxycarbonyl)benzoate, α-naphthoate, nitrate, alkyl *N,N,N',N'*-tetramethylphosphorodiamidate, and 2-chlorobenzoate. In the case of sulfonates, sulfenates and sulfinates the protecting group for the OH together with the oxygen atom of the unprotected OH to which it is attached form a sulfonate, sulfenate or sulfinates that can be selected from sulfate, allylsulfonate, methanesulfonate, benzylsulfonate, tosylate, 2-[(4-nitrophenyl)ethyl]sulfonate, 2-trifluoromethylbenzenesulfonate, 4-monomethoxytritylsulfenate, alkyl 2,4-dinitrophenylsulfenate, 2,2,5,5-tetramethylpyrrolidin-3-one-1-sulfinate, and dimethylphosphinothioyl. In the case of carbonates the protecting group for the OH together with the oxygen atom of the unprotected OH to which it is attached form a carbonate that can be selected from methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(phenylsulfonyl)ethyl carbonate, 2-(triphenylphosphonio)ethyl carbonate, *cis*-[4-[[(methoxytrityl)sulfenyl]oxy]tetrahydrofuran-3-yl]oxy carbonate, isobutyl carbonate, *t*-butyl carbonate, vinyl carbonate, allyl carbonate, cinnamyl carbonate, propargyl carbonate, p-chlorophenyl carbonate, *p*-nitrophenyl carbonate, 4-ethoxy-1-naphthyl carbonate, 6-bromo-7-hydroxycoumarin-4-ylmethyl carbonate, benzyl carbonate, *o*-nitrobenzyl carbonate, *p-*nitrobenzyl carbonate, *p*-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, 2-(3,4-methylenedioxy-6-nitrophenyl)propyl carbonate, 2-cyano-1-phenylethyl carbonate, 2-(2-pyridyl)amino-1-phenylethyl carbonate, 2-[*N*-methyl-*N*-(2-pyridyl)]amino-1-phenylethyl carbonate, phenacyl carbonate, 3',5'-dimethoxybenzoin carbonate, methyl dithiocarbonate, and *S*-benzyl thiocarbonate. And in the case of carbamates the protecting group for OH together with the oxygen atom of the unprotected OH to which it is attached forms a carbamate that can be selected from dimethyl thiocarbamate, *N*-phenyl carbamate, and *N*-methyl-*N*-(*o*-nitrophenyl) carbamate.

Within the scope of the present disclosure an amino protecting group is defined to be the *N*-bonded moiety resulting from the protection of the amino group through the formation of a suitable protected amino group. Examples of protected amino groups include carbamates, ureas, amides, heterocyclic systems, *N*-alkyl amines, *N*-alkenyl amines, *N*-alkynyl amines, *N*-aryl amines, imines, enamines, N-metal derivatives, N-N derivatives, N-P derivatives, N-Si derivatives, and N-S derivatives. In the case of carbamates the protecting group for the amino group together with the amino group to which it is attached form a carbamate that can be selected from methyl carbamate, ethyl carbamate, 9-fluorenylmethyl carbamate, 2,6-di-*t*-butyl-9-fluorenylmethyl carbamate, 2,7-bis(trimethylsilyl)fluorenylmethyl carbamate, 9-(2-sulfo)fluorenylmethyl carbamate, 9-(2,7-dibromo)fluorenylmethyl carbamate, 17-tetrabenzo[*a,c,g,i*]fluorenylmethyl carbamate, 2-chloro-3-indenylmethyl carbamate, benz[*f*]inden-3-ylmethyl carbamate, 1,1-dioxobenzo[b]-thiophene-2-ylmethyl carbamate, 2-methylsulfonyl-3-phenyl-1-prop-2-enyl carbamate, 2,7-di-t-butyl-[9,(10,10-dioxo-10,10,10,10-tetrahydrothioxanthyl)]methyl carbamate, 2,2,2-trichloroethyl carbamate, 2-trimethylsilylethyl carbamate, (2-phenyl-2-trimethylsilyl)ethyl carbamate, 2-phenylethyl carbamate, 2-chloroethyl carbamate, 1,1-dimethyl-2-haloethyl carbamate, 1,1-dimethyl-2,2-dibromoethyl carbamate, 1,1-dimethyl-2,2,2-trichloroethyl carbamate, 2-(2'-pyridyl)ethyl carbamate, 2-(4'-pyridyl)ethyl carbamate, 2,2-bis(4'-nitrophenyl)ethyl carbamate, 2-[(2-nitrophenyl)dithio]-1-phenylethyl carbamate, 2-(*N*,*N*-dicyclohexylcarboxamido)ethyl carbamate, *t*-butyl carbamate, fluorous BOC carbamate, 1-adamantyl carbamate, 2-adamantyl carbamate, 1-(1-adamantyl)-1-methylethyl carbamate, 1-methyl-1-(4-byphenylyl)ethyl carbamate, 1-(3,5-di-*t*-butylphenyl)-1-methylethyl carbamate, triisopropylsilyloxy carbamate, vinyl carbamate, allyl carbamate, prenyl carbamate, 1-isopropylallyl carbamate, cinnamyl carbamate, 4-nitrocinnamyl carbamate, 3-(3'-pyridyl)prop-2-enyl carbamate, hexadienyl carbamate, propargyl carbamate, 1,4-but-2-ynyl biscarbamate, 8-quinolyl carbamate, *N*-hydroxypiperidinyl carbamate, alkyl dithiocarbamate, benzyl carbamate, 3,5-di-*t*-butylbenzyl carbamate, *p*-methoxybenzyl carbamate, *p*-nitrobenzyl carbamate, *p-*bromobenzyl carbamate, *p*-chlorobenzyl carbamate, 2,4-dichlorobenzyl carbamate, 4-methylsulfinylbenzyl carbamate, 4-trifluoromethylbenzyl carbamate, fluorous benzyl carbamate, 2-naphthylmethyl carbamate, 9-anthrylmethyl carbamate, diphenylmethyl carbamate, 4-phenylacetoxybenzyl carbamate, 4-azidobenzyl carbamate, 4-azido-methoxybenzyl carbamate, *m*-chloro-*p*-acyloxybenzyl carbamate, *p*-(dihydroxyboryl)-benzyl carbamate, 5-benzisoxazolylmethyl carbamate, 2-(trifluoromethyl)-6-chromonylmethyl carbamate, 2-methylthioethyl carbamate, 2-methylsulfonylethyl carbamate, 2-(*p*-toluenesulfonyl)ethyl carbamate, 2-(4-nitrophenylsulfonyl)ethyl carbamate, 2-(2,4-dinitrophenylsulfonyl)ethyl carbamate, 2-(4-trifluoromethylphenylsulfonyl)ethyl carbamate, [2-(1,3-dithianyl)]methyl carbamate, 2-phosphonioethyl carbamate, 2-[phenyl(methyl)sulfonio]ethyl carbamate, 1-methyl-1-(triphenylphosphonio)ethyl carbamate, 1,1-dimethyl-2-cyanoethyl carbamate, 2-dansylethyl carbamate, 2-(4-nitrophenyl)ethyl carbamate, 4-methylthiophenyl carbamate, 2,4-dimethylthiophenyl carbamate, *m*-nitrophenyl carbamate, 3,5-dimethoxybenzyl carbamate, 1-methyl-1-(3,5-dimethoxyphenyl)ethyl carbamate, *α*-methylnitropiperonyl carbamate, *o-*nitrobenzyl carbamate, 3,4-dimethoxy-6-nitrobenzyl carbamate, phenyl(*o*-nitrophenyl)methyl carbamate, 2-nitrophenylethyl carbamate, 6-nitroveratryl carbamate, 4-methoxyphenacyl carbamate, 3',5'-dimethoxybenzoin carbamate, 9-xanthenylmethyl carbamate, *N*-methyl*-N*-(*o-*nitrophenyl) carbamate, *t*-amyl carbamate, 1-methylcyclobutyl carbamate, 1-methylcyclohexyl carbamate, 1-methyl-1-cyclopropylmethyl carbamate, cyclobutyl carbamate, cyclopentyl carbamate, cyclohexyl carbamate, isobutyl carbamate, isobornyl carbamate, cyclopropylmethyl carbamate, *p*-decyloxybenzyl carbamate, diisopropylmethyl carbamate, 2,2-dimethoxy-carbonylvinyl carbamate, *o*-(N,N-dimethylcarboxamido)benzyl carbamate, 1,1-dimethyl-3-(*N*,*N-*dimethyl-carboxamido)propyl carbamate, butynyl carbamate, 1,1-dimethylpropynyl carbamate, 2-iodoethyl carbamate, 1-methyl-1-(4'-pyridyl)ethyl carbamate, 1-methyl-1-(*p-*phenylazophenyl)ethyl carbamate, *p-*(*p*'-methoxyphenylazo)benzyl carbamate, *p-*(phenylazo)benzyl carbamate, 2,4,6-trimethylbenzyl carbamate, isonicotinyl carbamate, 4-(trimethyl-ammonium)benzyl carbamate, *p*-cyanobenzyl carbamate, di(2-pyridyl)methyl carbamate, 2-furanylmethyl carbamate, phenyl carbamate, 2,4,6-tri-*t*-butylphenyl carbamate, 1-methyl-1-phenylethyl carbamate, and *S*-benzyl thiocarbamate. In the case of ureas the protecting groups for the amino group can be selected from phenothiazinyl-(10)-carbonyl, *N'-p-*toluenesulfonylaminocarbonyl, *N*'-phenylaminothiocarbonyl, 4-hydroxyphenylaminocarbonyl, 3-hydroxytryptaminocarbonyl, and *N*'-phenylaminothiocarbonyl. In the case of amides the protecting group for the amino together with the amino group to which it is attached form an amide that can be selected from formamide, acetamide, chloroacetamide, trichloroacetamide, trifluoroacetamide, phenylacetamide, 3-phenylpropanamide, pent-4-enamide, picolinamide, 3-pyridylcarboxamide, *N*-benzoylphenylalanyl amide, benzamide, *p*-phenylbenzamide, *o-*nitrophenylacetamide, 2,2-dimethyl-2-(*o*-nitrophenyl)acetamide, *o*-nitrophenoxyacetamide, 3-(*o*-nitrophenyl)propanamide, 2-methyl-2-(*o*-nitrophenoxy)propanamide, 3-methyl-3-nitrobutanamide, *o*-nitrocinnamide, *o*-nitrobenzamide, 3-(4-*t*-butyl-2,6-dinitrophenyl)-2,2-dimethylpropanamide, o-(benzoyloxyme-thyl)benzamide, 2-(acetoxymethyl)benzamide, *2*-[(t-butyldiphenylsiloxy)methyl]benzamide, 3-(3',6'-dioxo-2',4',5'-trimethylcyclohexa-1',4'-diene)-3,3-dimethylpropionamide, *o*-hydroxy-*trans*-cinnamide, 2-methyl-2-(*o-*phenylazophenoxy)propanamide, 4-chlorobutanamide, aceto-acetamide, *3*-(*p-*hydroxyphenyl)propanamide, (*N'*-dithiobenzyloxycarbonylamino)acetamide, and *N-*acetylmethionine amide. In the case of heterocyclic systems the protecting group for the amino group together with the amino group to which it is attached form a heterocyclic system that can be selected from 4,5-diphenyl-3-oxazolin-2-one, *N*-phthalimide, *N*-dichlorophthalimide, *N-*tetrachlorophthalimide, *N*-4-nitrophthalimide, *N*-thiodiglycoloyl, *N*-dithiasuccinimide, *N*-2,3-diphenylmaleimide, *N*-2,3-dimethylmaleimide, *N*-2,5-dimethylpyrrole, *N*-2,5-bis(triisopropylsiloxy)pyrrole, *N-*1,1,4,4-tetramethyldisilylazacyclopentane adduct, *N*-1,1,3,3-tetramethyl-1,3-disilaisoindoline,*N*-diphenylsilyldiethylene, *N*-5-substituted-1,3-dimethyl-1,3,5-triazacyclohexan-2-one, *N*-5-substituted-1,3-benzyl-1,3,5-triazacyclohexan-2-one, 1-substituted 3,5-dinitro-4-pyridone, and 1,3,5-dioxazine. In the case of *N*-alkyl, *N*-alkenyl, *N*-alkynyl or *N-*aryl amines the protecting group for the amino group can be selected from *N*-methyl, *N-t*-butyl, *N*-allyl, *N*-prenyl, *N*-cinnamyl, *N*-phenylallyl, *N*-propargyl, *N*-methoxymethyl, *N*-[2-(trimethylsilyl)ethoxy]methyl, *N*-3-acetoxypropyl, *N*-cyanomethyl, *N*-2-azanorbornenes, *N-*benzyl, *N*-4-methoxybenzyl, *N*-2,4-dimethoxybenzyl, *N*₋2-hydroxybenzyl, *N*-ferrocenylmethyl, *N*-2,4-dinitrophenyl, o-methoxyphenyl, p-methoxyphenyl, *N*-9-phenylfluorenyl, *N*-fluorenyl, *N-*2-picolylamine N'-oxide, *N-*7-methoxycoumar-4-ylmethyl, *N*-diphenylmethyl, *N*-bis(4-methoxyphenyl)methyl, *N*-5-dibenzosuberyl, *N*-triphenylmethyl, *N*-(4-methylphenyl)diphenylmethyl, and *N*-(4-methoxyphenyl)diphenylmethyl. In the case of imines the protecting group for the amino group can be selected from *N*-1,1-dimethylthiomethylene, *N-*benzylidene, *N-p*-methoxybenzylidene, *N*-diphenylmethylene, *N-*[2-pyridyl)mesityl]methylene, *N-*(*N*',*N*'-dimethylaminomethylene), *N-*(*N'*,*N'-*dibenzylaminomethylene), *N-*(*N'-t-*butylaminome-thylene), *N*,*N*-isopropylidene, *N*-*p*-nitrobenzylidene, *N*-salicylidene, *N*-5-chlorosalicylidene, *N-*(5-chloro-2-hydroxyphenyl)phenylmethylene,*N*-cyclohexylidene, and *N*-*t*-butylidene. In the case of enamines the protecting group for the amino group can be selected from *N*-(5,5-dimethyl-3-oxo-1-cyclohexenyl), *N*-2,7-dichloro-9-fluorenylmethylene, *N*-1-(4,4-dimethyl-2,6-dioxocyclohexylidene)ethyl, *N*-(1,3-dimethyl-2,4,6-(1*H*,3*H*,5*H*)-trioxopyrimidine-5-ylidene)-methyl, *N*-4,4,4-trifluoro-3-oxo-1-butenyl, and *N-*(1-isopropyl-4-nitro-2-oxo-3-pyrrolin-3-yl). In the case of N-metal derivatives the protecting group for the amino group can be selected from N-borane, *N*-diphenylborinic ester, *N*-diethylborinic ester, *N*-9-borabicyclononane, *N-*difluoroborinic ester, and 3,5-bis(trifluoromethyl)phenylboronic acid; and also including *N-*phenyl(pentacarbonylchromium)carbenyl, *N*-phenyl(pentacarbonyl-tungsten)carbenyl, *N-*methyl(pentacarbonylchromium)carbenyl, *N*-methyl(pentacarbonyltungsten)carbenyl, N-copper chelate, *N*-zinc chelate, and a 18-crown-6-derivative. In the case of N-N derivatives the protecting group for the amino group together with the amino group to which it is attached form a N-N derivative that can be selected from *N*-nitroamino, *N*-nitrosoamino, amine *N*-oxide, azide, triazene derivative, and *N*-trimethylsilylmethyl-*N*-benzylhydrazine. In the case of N-P derivatives the protected group for the amino group together with the amino group to which it is attached form a N-P derivative that can be selected from diphenylphosphinamide, dimethylthiophosphinamide, diphenylthiophosphinamide, dialkyl phosphoramidate, dibenzyl phosphoramidate, diphenyl phosphoramidate, and iminotriphenylphosphorane. In the case of N-Si derivatives the protecting group for the NH₂ can be selected from *t*-butyldiphenylsilyl and triphenylsilyl. In the case of N-S derivatives the protected amino group can be selected from N-sulfenyl or N-sulfonyl derivatives. The N-sulfenyl derivatives can be selected from benzenesulfenamide, 2-nitrobenzenesulfenamide, 2,4-dinitrobenzenesulfenamide, pentachlorobenzenesulfenamide, 2-nitro-4-methoxybenzenesulfenamide, triphenylmethylsulfenamide, 1-(2,2,2-trifluoro-1,1-diphenyl)ethylsulfenamide, and *N*-3-nitro-2-pyridinesulfenamide. The N-sulfonyl derivatives can be selected from methanesulfonamide, trifluoromethanesulfonamide, *t*-butylsulfonamide, benzylsulfonamide, 2-(trimethylsilyl) ethanesulfonamide, *p*-toluenesulfonamide, benzenesulfonamide, *o*-anisylsulfonamide, 2-nitrobenzenesulfonamide, 4-nitrobenzenesulfonamide, 2,4-dinitrobenzenesulfonamide, 2-naphthalene sulfonamide, 4-(4',8'-dimethoxynaphthylmethyl)benzenesulfonamide, 2-(4-methylphenyl)-6-methoxy-4-methylsulfonamide, 9-anthracenesulfonamide, pyridine-2-sulfonamide, benzothiazole-2-sulfonamide, phenacylsulfonamide, 2,3,6-trimethyl-4-methoxybenzenesulfonamide, 2,4,6-trimethoxybenzenesulfonamide, 2,6-dimethyl-4-methoxybenzenesulfonamide, pentamethylbenzenesulfonamide, 2,3,5,6-tetramethyl-4-methoxybenzenesulfonamide, 4-methoxybenzenesulfonamide, 2,4,6-trimethylbenzenesulfonamide, 2,6-dimethoxy-4-methylbenzenesulfonamide, 3-methoxy-4-t-butylbenzenesulfonamide, and 2,2,5,7,8-pentamethylchroman-6-sulfonamide.

The mention of these groups should not be interpreted as a limitation of the scope of the invention, since they have been mentioned as a mere illustration of protecting groups for OH groups, but further groups having said function may be known by the skilled person in the art, and they are to be understood to be also encompassed by the present invention.

Examples of suitable reagents for the synthesis of carbamates include, but are not limited to, trichloroacetyl isocyanate, trimethylsilyl isocyanate and triphenylsilyl isocyanate.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

The present invention is directed to a compound of formula I: 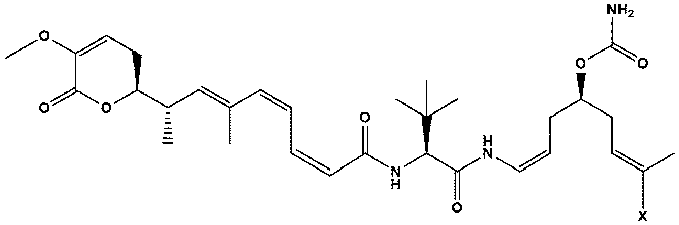 wherein X = F or Me.

The present invention is also directed to a compound of formula II:

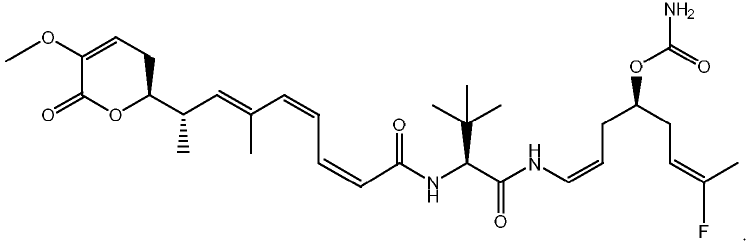

The present invention is also directed to a compound of formula III: 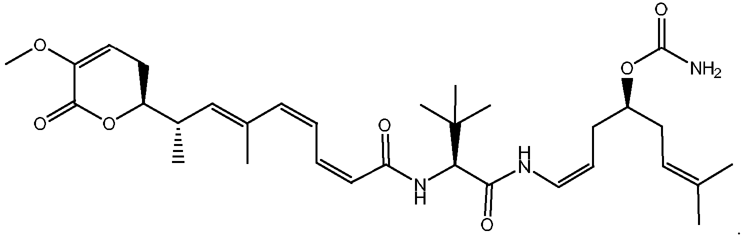 An important feature of the above-described compounds is their bioactivity and in particular their cytotoxic activity. In this regard, it has surprisingly been found that the compounds of the present invention show an enhanced antitumor activity versus prior art compounds PM060184 and PM050489. This is shown for PM060184 in the *in vitro* bioassays in Example 3 where it can be seen that 2 and 3 both have surprisingly improved *in vitro* activity versus prior art compound PM060184. 2 is between 3.8 to 8.2 times more active than PM060184; and 3 is between 1.3 to 3.7 times more active than PM060184. This is seen across A549, HT29, MDA-MB-231 and PSN-1 cell lines. This is also shown in the *in vivo* xenograft models in Examples 5-7 where 2 and 3 show a remarkable improvement in efficacy over PM060184 across a wide range of cancer models. In H460, HGC27 and HCT116 xenograft models (which model non-small cell lung cancer, gastric cancer and colorectal carcinoma), **2** and **3** both show statistically significant improvements not only in antitumor activity but also in survival time across all three cell lines.
This is also shown for PM050489 in the *in vitro* bioassays in Example 3 where it can be seen that **2** and **3** both have improved *in vitro* activity versus prior art compound PM050489. Compound 2 is between 1.9 to 7.9 times more active than reference compound PM050489. Compound 3 is between 1.3 to 1.8 times more active than reference compound PM050489. This is seen across A549, HT29, MDA-MB-231 and PSN-1 cell lines. This is also shown in the *in vivo* xenograft models, where there is a significant improvement in efficacy over PM050489 in the H460 xenograft model. This is seen from Example 3 for *in vitro* data and from the Comparative Example and Example 5 for *in vivo* data.
In a further embodiment of the present invention, there is provided a pharmaceutical composition comprising a compound according to the present invention and a pharmaceutically acceptable carrier. Examples of the administration form include without limitation oral, topical, parenteral, sublingual, rectal, vaginal, ocular and intranasal. Parenteral administration includes subcutaneous injections, intravenous, intramuscular, intrasternal injection or infusion techniques. Preferably the compositions are administered parenterally. Pharmaceutical compositions of the invention can be formulated so as to allow a compound according to the present invention to be bioavailable upon administration of the composition to an animal, preferably human. Compositions can take the form of one or more dosage units, where for example, a tablet can be a single dosage unit, and a container of a compound according to the present invention may contain the compound in liquid or in aerosol form and may hold a single or a plurality of dosage units.

The pharmaceutically acceptable carrier or vehicle can be particulate, so that the compositions are, for example, in tablet or powder form. The carrier(s) can be liquid, with the compositions being, for example, an oral syrup or injectable liquid. In addition, the carrier(s) can be gaseous, or liquid so as to provide an aerosol composition useful in, for example inhalatory administration. Powders may also be used for inhalation dosage forms. The term "carrier" refers to a diluent, adjuvant or excipient, with which the compound according to the present invention is administered. Such pharmaceutical carriers can be liquids, such as water and oils including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, disaccharides, cyclodextrins, cyclodextrin derivatives and the like. In addition, auxiliary, stabilizing, thickening, lubricating and coloring agents can be used. In one embodiment, when administered to an animal, the compounds and compositions according to the present invention, and pharmaceutically acceptable carriers are sterile. Water is a preferred carrier when the compounds according to the present invention are administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, cyclodextrins, cyclodextrin derivatives, glycerol, propylene glycol, water, ethanol and the like. The present compositions, if desired, can also contain minor amounts of wetting or emulsifying agents, or pH buffering agents.

When intended for oral administration, the composition is preferably in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more for the following can be present: binders such as carboxymethylcellulose, ethyl cellulose, microcrystalline cellulose, or gelatin; excipients such as starch, cyclodextrins, cyclodextrin derivatives, lactose or dextrins, disintegrating agents such as alginic acid, sodium alginate, corn starch and the like; lubricants such as magnesium stearate; glidants such as colloidal silicon dioxide; sweetening agent such as sucrose or saccharin; a flavoring agent such as peppermint, methyl salicylate or orange flavoring; and a coloring agent.

When the composition is in the form of a capsule (e.g. a gelatin capsule), it can contain, in addition to materials of the above type, a liquid carrier such as polyethylene glycol, cyclodextrins or a fatty oil.

The composition can be in the form of a liquid, e.g. an elixir, syrup, solution, emulsion or suspension. The liquid can be useful for oral administration or for delivery by injection. When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included.

The preferred route of administration is parenteral administration including, but not limited to, intradermal, intramuscular, intraperitoneal, intravenous, subcutaneous, intranasal, epidural, intracerebral, intraventricular, intrathecal, intravaginal or transdermal. The preferred mode of administration is left to the discretion of the practitioner, and will depend in part upon the site of the medical condition (such as the site of cancer). In a more preferred embodiment, the compounds according to the present invention are administered intravenously. Infusion times of up to 24 hours are preferred to be used, more preferably 1 to 12 hours, with 1 to 6 hours being most preferred. Short infusion times which allow treatment to be carried out without an overnight stay in a hospital are especially desirable. However, infusion may be 12 to 24 hours or even longer if required. Infusion may be carried out at suitable intervals of, for example, 1 to 4 weeks.

The liquid compositions of the invention, whether they are solutions, suspensions or other like form, can also include one or more of the following: sterile diluents such as water for injection, saline solution, preferably physiological saline, Ringer's solution, isotonic sodium chloride, fixed oils such as synthetic mono or diglycerides, polyethylene glycols, glycerin, or other solvents; antibacterial agents such as benzyl alcohol or methyl paraben; and agents for the adjustment of tonicity such as sodium chloride or dextrose. A parenteral composition can be enclosed in an ampoule, a disposable syringe or a multiple-dose vial made of glass, plastic or other material. Physiological saline is a preferred adjuvant.

The amount of the compound according to the present invention that is effective in the treatment of a particular disorder or condition will depend on the nature of the disorder or condition, and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgement of the practitioner and each patient's circumstances.

The compositions comprise an effective amount of a compound of the present invention such that a suitable dosage will be obtained. The correct dosage of the compounds will vary according to the particular formulation, the mode of application, and its particular site, host and the disease being treated, e.g. cancer and, if so, what type of tumor. Other factors like age, body weight, sex, diet, time of administration, rate of excretion, condition of the host, drug combinations, reaction sensitivities and severity of the disease should be taken into account. Administration can be carried out continuously or periodically within the maximum tolerated dose.

Typically, the amount is at least about 0.01% of a compound of the present invention, and may comprise at least 80%, by weight of the composition. When intended for oral administration, this amount can be varied to range from about 0.1% to about 80% by weight of the composition. Preferred oral compositions can comprise from about 4% to about 50% of the compound of the present invention by weight of the composition.

Preferred compositions of the present invention are prepared so that a parenteral lyophilized dosage unit contains from about 0.005% to about 10 % by weight of the compound of the present invention.

For intravenous administration, exemplary doses include a total dose per cycle of between about 10.5 mg/m² to about 84 mg/m² (or about 10 mg/m² to about 85 mg/m²). The total dose per cycle may be split according to the dosing schedule. Exemplary doses include a daily dose range of about about 0.5 mg/m²/day to about 3 mg/m²/day. Daily dose values may be consolidated according to the dosing schedule. In order to equate a particular dosing schedule to the exemplary doses, the specfic doses administered over the course of the cycle are added to provide the total dose per cycle, and dividing this total dose per cycle by the number of days in the cycle gives the daily dose.

The compound of the present invention, can be administered by any convenient route, for example by infusion or bolus injection, by absorption through epithelial or mucocutaneous linings.

In specific embodiments, it can be desirable to administer one or more compounds of the present invention, or compositions locally to the area in need of treatment. In one embodiment, administration can be by direct injection at the site (or former site) of a cancer, tumor or neoplastic or pre-neoplastic tissue.

Pulmonary administration can also be employed, e.g. by use of an inhaler or nebulizer, and formulation with an aerosolizing agent, or via perfusion in a fluorocarbon or synthetic pulmonary surfactant. In certain embodiments, the compound of the present invention can be formulated as a suppository, with traditional binders and carriers such as triglycerides.

The present compositions can take the form of solutions, suspensions, emulsions, tablets, pills, pellets, capsules, capsules containing liquids, powders, sustained-release formulations, suppositories, emulsions, aerosols, sprays, suspensions, or any other form suitable for use. Other examples of suitable pharmaceutical carriers are described in "Remington's Pharmaceutical Sciences" by E. W. Martin.
The pharmaceutical compositions can be prepared using methodology well known in the pharmaceutical art. For example, a composition intended to be administered by injection can be prepared by combining a compound of the present invention with water, or other physiologically suitable diluent, such as phosphate buffered saline, so as to form a solution. A surfactant can be added to facilitate the formation of a homogeneous solution or suspension.

The composition comprising a compound of the present invention may be lyophilized. The composition comprising a compound of the present invention is usually presented in a vial which contains a specified amount of such compound.

We have found that the compounds of the present invention and compositions of the present invention are particularly effective in the treatment of cancer.

Thus, as described earlier, the present invention provides a method of treating a patient in need thereof, notably a human, affected by cancer which comprises administering to the affected individual a therapeutically effective amount of a compound or composition according to the present invention. The present invention provides a compound or composition for use as medicament. The present invention provides a compound or composition for use in the treatment of cancer, and more preferably a solid tumor, yet more preferably a cancer selected from lung cancer, non-small cell lung cancer, colorectal cancer, breast cancer, pancreatic cancer and gastric cancer.

Thus, the compounds and compositions according to the present invention are useful for inhibiting the multiplication, or proliferation, of a tumor cell or cancer cell, or for treating cancer in an animal.

The compounds and compositions according to the present invention show excellent activity in the treatment of cancers, notably solid tumors, such as lung cancer, non-small cell lung cancer, colorectal cancer, breast cancer, pancreatic cancer and gastric cancer.

In the present application, by "cancer" it is meant to include tumors, neoplasias and any other malignant disease having as cause malignant tissue or cells.

The term "treating", as used herein, unless otherwise indicated, means reversing, attenuating, alleviating or inhibiting the progress of the disease or condition to which such term applies, or one or more symptoms of such disorder or condition. The term "treatment", as used herein, unless otherwise indicated, refers to the act of treating as "treating" is defined immediately above.

The compounds and compositions according to the present invention can be administered to an animal that has also undergone surgery as treatment for the cancer. In one embodiment of the present invention, the additional method of treatment is radiation therapy.

In a specific embodiment of the present invention, the compound or composition according to the present invention is administered concurrently with radiation therapy. In another specific embodiment, the radiation therapy is administered prior or subsequent to administration of the compound or composition of the present invention, preferably at least an hour, three hours, five hours, 12 hours, a day, a week, a month, more preferably several months (e.g. up to three months) prior or subsequent to administration of a compound or composition of the present invention.

Any radiation therapy protocol can be used depending upon the type of cancer to be treated. For example, but not by way of limitation, x-ray radiation can be administered; in particular, high-energy megavoltage (radiation of greater than 1 MeV energy) can be used for deep tumors, and electron beam and orthovoltage x-ray radiation can be used for skin cancers. Gamma-ray emitting radioisotopes, such as radioactive isotopes of radium, cobalt and other elements, can also be administered.

In a further embodiment of the present invention, there is provided a kit comprising a therapeutically effective amount of a compound according to the present invention and a pharmaceutically acceptable carrier.

In one embodiment, the kit according to this embodiment is for use in the treatment of cancer, more preferably a solid tumor, and more preferably a cancer selected from lung cancer, non-small cell lung cancer, colorectal cancer, breast cancer, pancreas cancer, and gastric cancer.

In a further embodiment of the present invention, there is provided a process for the manufacture of a compound according to the present invention, the process comprising reacting a compound of formula **V** with a suitable reagent for the synthesis of carbamates to give a compound of formula **I** 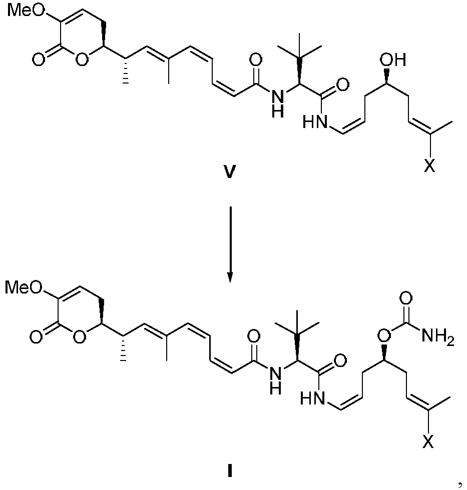 wherein X is F or Me.

In another embodiment, the process further comprises the coupling of a compound of formula **VI** with a compound of formula **VII,** wherein R₃ is an unsubstituted C₁-C₄ alkyl group, ProtOH is a protecting group for hydroxy and X is as defined for formula **I**; followed by deprotecting the OProt^{OH} group to give a compound of formula **V.** In an embodiment, there is provided a process to manufacture a compound of formula **I** comprising coupling a compound of formula **VI** with a compound of formula **VII,** wherein R₃ is an unsubstituted C₁-C₄ alkyl group, ProtOH is a protecting group for hydroxy and X is as defined for formula **I;** followed by deprotecting the OProt^{OH} group to give a compound of formula **V,** and converting the compound of formula **V** into a compound of formula **I.**

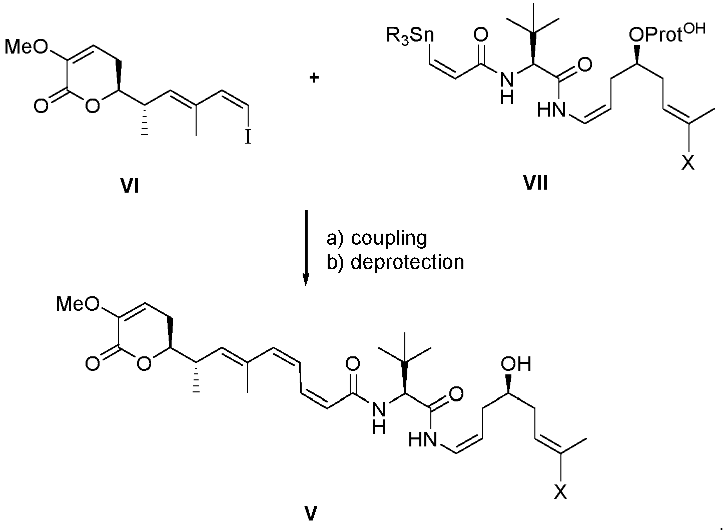

In another embodiment, the process further comprises the coupling of a compound of formula **VIII** wherein R is as defined for formula **VII,** with a compound of formula **IX** wherein Prot^{OH} is a protecting group for hydroxy and X is as defined for formula **I** to give a compound of formula **VII.** In an embodiment, there is provided a process to manufacture a compound of formula **I** comprising coupling a compound of formula **VIII** wherein R is as defined for formula **VII,** with a compound of formula **IX** wherein Prot^{OH} is a protecting group for hydroxy and X is as defined for formula **I** to give a compound of formula **VII,** and converting the compound of formula **VII** into a compound of formula **I.**

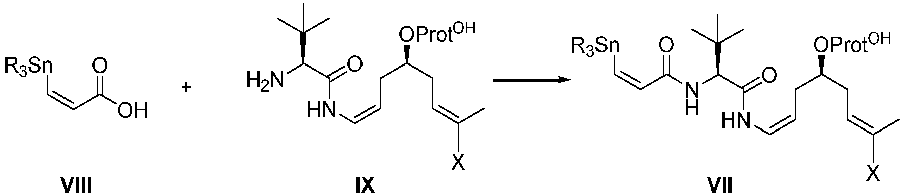

In another embodiment, the process further comprises the coupling of a compound of formula **X** where Prot^{NH} is a protecting group for amino with a compound of formula **XI** wherein ProtOH is a protecting group for hydroxy and X is as defined for formula **I,** followed by deprotecting the NHProt^{NH} group to give a compound of formula **IX.** In an embodiment, there is provided a process to manufacture a compound of formula **I** comprising coupling compound of formula **X** where Prot^{NH} is a protecting group for amino with a compound of formula **XI** wherein ProtOH is a protecting group for hydroxy and X is as defined for formula **I,** followed by deprotecting the NHProt^{NH} group to give a compound of formula **IX,** and converting the compound of formula **IX** into a compound of formula **I.**

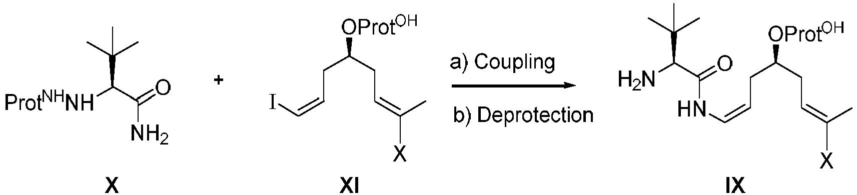

In a further embodiment of the present invention there are provided intermediates of formula **V, VII, IX** or **XI** useful for the manufacture of compounds of formula I. In a preferred embodiment an intermediate of formula **V** is provided.

### EXAMPLES

Compounds **5** and **17** can be prepared as described in Example 6 and 5 respectively of WO 2007/144423.

Reference compounds **PM050489** and **PM060184** were prepared as described in WO 2007/144423 (Compounds **1** and **4,** respectively).

### EXAMPLE 1 - SYNTHESIS OF 2

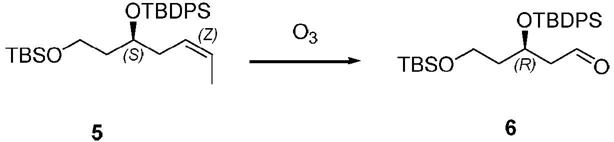

Ozone is bubbled through a solution of the alkene **5** (comp **22b** in WO2007144423) (113.5 g, 235 mmol) and anhydrous pyridine (28.5 mL, 352.6 mmol) in CH₂Cl₂ (1175 mL) at -78 °C until the solution takes on blue-green color. Ozone is purged with N₂ for 30 minutes and the mixture is filtered through a silica pad (1200 g) in a Buchner funnel eluting with CH₂Cl₂ to obtain **6** (75.3 g, 68% yield) as a colourless liquid.

¹H NMR (400 MHz, CDCl₃): δ 9.67 (dd, *J* = 2.9, 2.1 Hz, 1H), 7.70-7.57 (m, 4H), 7.50-7.33 (m, 6H), 4.39 (dq, *J* = 6.8, 5.6 Hz, 1H), 3.63 (ddd, *J* = 10.5, 7.1, 5.6 Hz, 1H), 3.55 (dt, *J* = 10.4, 6.0 Hz, 1H), 2.58 (ddd, *J* = 15.9, 5.6, 2.1 Hz, 1H), 2.48 (ddd, *J* = 16.0, 5.7, 2.9 Hz, 1H), 1.84 (ddt, *J* = 13.8, 7.0, 5.7 Hz, 1H), 1.78-1.66 (m, 1H), 1.05 (s, 9H), 0.82 (s, 9H), 0.04 (d, *J=* 4.9 Hz, 6H). ESI-MS *m*/*z:* 493.3 [M+Na]⁺.

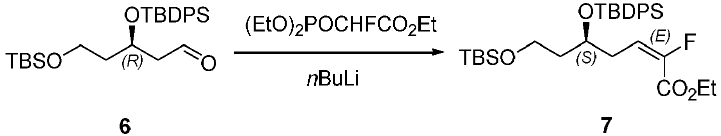

To a solution of Ethyl 2-(diethoxyphosphoryl)-2-fluoroacetate (35.8 mL, 176 mmol) in THF (1280 mL) at -78 °C was slowly added nBuLi (110 mL, 1.6 M in hexanes, 176 mmol). After stirring for 30 min, a solution of **6** (75.3 g, 160 mmol) in THF (320 mL) cooled at -78 °C, was then added in 55 min. The reaction mixture was stirred at -78 °C for 2.5 h and quenched by addition of an aqueous saturated solution of NH₄Cl (1.16 L). The mixture was diluted with H₂O (170 mL), EtOAc (440 mL) was added and the layers were separated. The aqueous layer was extracted with EtOAc (200 mL) and the combined organic layers were dried over anhydrous Na₂SO₄, filtered and concentrated to dryness. This crude was purified by flash chromatography (Hex:EtOAc 99:1) to obtain 7 (70.5 g, 79% yield) as a colourless oil.

¹H NMR (400 MHz, CDCl₃): δ 7.71-7.60 (m, 4H), 7.49-7.28 (m, 6H), 5.96 (ddd, *J =* 21.9, 8.6, 6.9 Hz, 1H), 4.23 (q, *J* = 7.1 Hz, 2H), 4.03 (q, *J* = 5.6 Hz, 1H), 3.65-3.45 (m, 2H), 2.83-2.45 (m, 2H), 1.86-1.70 (m, 1H), 1.67-1.58 (m, 1H), 1.30 (t, *J* = 7.2 Hz, 3H), 1.05 (s, 9H), 0.81 (s, 9H), 0.02 (s, 6H).

¹⁹F-NMR (376 MHz, CDCl₃): δ -120.3 (dd, *J* = 21.8, 2.8 Hz).

ESI-MS *m*/*z:* 581.2 [M+Na]⁺.

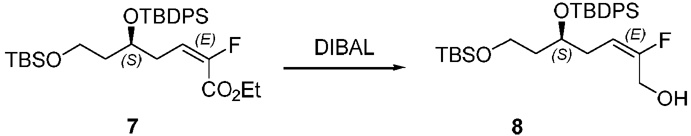

To a solution of 7 (70.3 g, 126 mmol) in anhydrous THF (754 mL) DIBAL (277 mL, 1.0 M in toluene, 277 mmol) was dropwise added in 30 min at 0 °C. The reaction mixture was stirred at 0 °C for 15 minutes and then, 3 hours at 23 °C. The reaction mixture was again cooled at 0 °C and quenched with MeOH (73 mL). Aqueous citric acid 10% solution (960 mL) was added and the mixture was stirred for 10 min and extracted with EtOAc (700 mL). The organic layer was dried over anhydrous Na₂SO₄, filtered and the solvent evaporated to dryness. This crude was purified by flash chromatography (Hex:EtOAc from 93:7 to 80:20) to afford 8 (64.68 g, 99% yield).

¹H NMR (400 MHz, CDCl₃): δ 7.71-7.63 (m, 4H), 7.48-7.33 (m, 6H), 5.13 (ddd, *J=* 21.0, 8.8, 7.6 Hz, 1H), 3.97-3.85 (m, 3H), 3.77-3.53 (m, 2H), 2.23-2.02 (m, 2H), 1.77-1.59 (m, 2H), 1.05 (s, 9H), 0.84 (s, 9H), 0.01 (s, 6H).

¹⁹F-NMR (376 MHz, CDCl₃): δ -111.2 (q, *J=* 21.0 Hz).

ESI-MS *m*/*z:* 539.3 [M+Na]⁺.

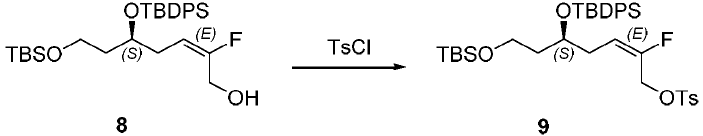

To a solution of **8** (26.55 g, 51 mmol) in anhydrous CH₂Cl₂ (460 mL) were consecutively added DMAP (0.25 g, 2 mmol), Et₃N (12.2 mL, 87 mmol), and finally *p*-Toluenesulfonyl chloride (12.7 g, 67 mmol) at 0 °C. The reaction mixture was stirred at 0 °C for 3 hours and quenched with H₂O (390 mL). Phases were separated and aqueous layer was extracted with CH₂Cl₂ (150 mL). The combined organic layers were dried over anhydrous Na₂SO₄, filtered and evaporated to dryness. The crude was purified by flash chromatography (Hex:EtOAc from 99:1 to 90:10) to yield pure 9 (26.7 g, 78% yield).

¹H NMR (400 MHz, CDCl₃): δ 7.78-7.70 (m, 2H), 7.66-7.61 (m, 4H), 7.48-7.37 (m, 2H), 7.41-7.30 (m, 4H), 7.34-7.23 (m, 2H), 5.34-5.20 (m, 1H), 4.42 (s, 1H), 4.37 (s, 1H), 3.88 (m, 1H), 3.65-3.48 (m, 2H), 2.46-2.41 (s, 3H), 2.15-1.95 (m, 2H), 1.74-1.50 (m, 2H), 1.03 (s, 9H), 0.84 (s, 9H), 0.01(s, 6H).

¹⁹F-NMR (376 MHz, CDCl₃): δ -109.6 (q, *J* = 20.3 Hz).

ESI-MS *m*/*z:* 693.3 [M+Na]⁺.

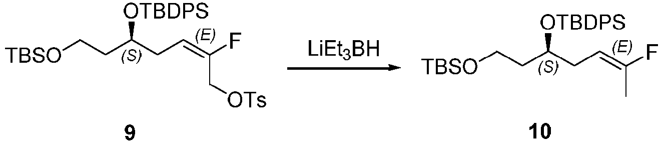

To a solution of **9** (26.65 g, 40 mmol) in anhydrous THF (260 mL) was slowly added, via addition funnel, LiEt₃BH (46.7 mL, 1.7 M in THF, 79 mmol) over a period of 20 minutes at 0 °C. After stirring for 2 additional hours, the reaction was carefully quenched with aqueous citric acid 10% solution (472 mL). The mixture was diluted with EtOAc (300 mL), followed by H₂O (100 mL) and the layers were separated. Aqueous layer was extracted with EtOAc (100 mL) and the combined organic layers were washed with brine, dried over anhydrous Na₂SO₄, filtered, and concentrated to dryness. The crude was filtered through a silica pad (Hex:EtOAc 90:10)to remove traces of boron salts and to obtain **10** (18.4 g, 93% yield) which was used in the next step without further purification.

¹H NMR (400 MHz, CDCl₃): δ 7.69-7.65 (m, 4H), 7.47-7.30 (m, 6H), 5.05-4.81 (m, 1H), 3.88 (p, *J=* 5.9 Hz, 1H), 3.70-3.55 (m, 2H), 2.14-1.89 (m, 2H), 1.76-1.56 (m, 5H), 1.05 (s, 9H), 0.84 (s, 9H), 0.01(s, 6H).

¹⁹F-NMR (376 MHz, CDCl₃): δ -93.9 (dq, *J =* 21.8, 17.6 Hz).

ESI-MS *m*/*z:* 523 [M+Na]⁺.

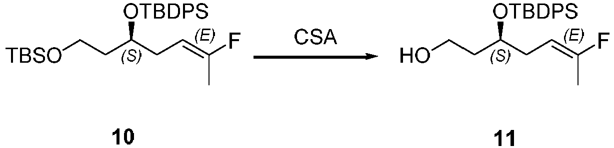

To a solution of **10** (18.3 g, 37 mmol) in CH₂Cl₂:CH₃OH (85:15 400 mL) Camphorsulfonic acid (3.4 g, 15 mmol) was added at 23 °C and the reaction mixture was stirred for 4 h at 23 °C. A saturated aqueous solution of NaHCO₃ (300 mL) was added (pH= 9) and the layers were separated. Aqueous layer was extracted with CH₂Cl₂ (100 mL), the combined organic layers were dried over anhydrous Na₂SO₄, filtered, concentrated and purified by flash chromatography (Hex:EtOAc from 100:0 to 50:50) to obtain pure 11 (13.56 g, 96% yield).

¹H NMR (400 MHz, CDCl₃): δ 7.78-7.63 (m, 4H), 7.50-7.32 (m, 6H), 4.91-4.73 (m, 1H), 3.96-3.89 (m, 1H), 3.81-3.62 (m, 2H), 2.22-1.94 (m, 2H), 1.89-1.76 (m, 1H), 1.69-1.56 (m, 4H), 1.06 (s, 9H).

¹⁹F-NMR (376 MHz, CDCl₃): δ -93.2 (dq, *J =* 21.7, 17.7 Hz).

ESI-MS *m*/*z:* 409.3 [M+Na]⁺.

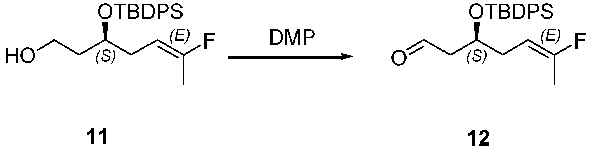

To a solution of **11** (13.3 g, 34 mmol) in anhydrous CH₂Cl₂ (210 mL) NaHCO₃ (16.0 g, 191 mmol) and Dess-Martin periodinane (16.0 g, 38 mmol) were successively added at 23 °C. The suspension was stirred for 1 h at 23 °C and cooled at 0 °C. A mixture of an aqueous saturated solution of Na₂S₂O₃ and an aqueous saturated solution of NaHCO₃ (50:50, 350 mL) was carefully added and stirred an additional hour. The mixture was diluted with H₂O (100 mL) and the layers were separated. Aqueous layer was extracted with CH₂Cl₂ (100 mL) and the combined organic layers were dried over anhydrous Na₂SO₄, filtered, and concentrated to dryness to afford crude **12** (12.35 g, 93% yield) which was used in the next step without further purification.

¹H NMR (400 MHz, CDCl₃): δ 9.70 (dd, *J* = 2.7, 2.0 Hz, 1H), 7.72-7.62 (m, 4H), 7.49-7.34 (m, 6H), 4.89 (dtd, *J* = 21.4, 8.1, 1.0 Hz, 1H), 4.23-4.16 (m, 1H), 2.58-2.42 (m, 2H), 2.15-2.09 (m, 2H), 1.66 (dq, *J* = 17.6, 0.9 Hz, 3H), 1.05 (s, 9H).

¹⁹F-NMR (376 MHz, CDCl₃): δ -91.9 (dq, *J* = 21.7, 17.3 Hz).

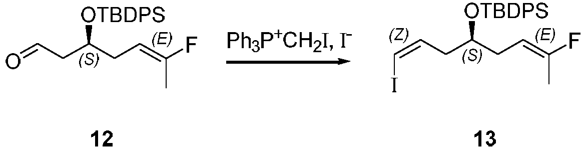

To a yellow suspension of iodomethylenetriphenylphosphorane (1.63 g, 3.1 mmol) in anhydrous THF (21 mL) NaHMDS (3.1 mL, 1.0 M in THF, 3.1 mmol) was dropwise added at 23 °C. After stirring for an additional 15 min, the solution was cooled to -78 °C and **12** (0.79 g, 2.1 mmol) in anhydrous THF (4 mL) was dropwise added. The temperature was kept at -78 °C while the reaction mixture was stirred for 2 hours. Hexane (45 mL) was added and the mixture filtered through a silica pad eluting with Hex:EtOAc 75:25. Evaporation of volatiles gave an oil that was purified by flash chromatography (Hex:EtOAc from 100:0 to 90: 10) to afford **13** (0.67 g, 64% yield).

¹H NMR (400 MHz, CDCl₃): δ 7.77-7.63 (m, 4H), 7.48-7.30 (m, 6H), 6.30-6.17 (m, 2H), 4.99-4.85 (m, 1H), 3.86 (p, *J =* 5.6 Hz, 1H), 2.35-2.24 (m, 2H), 2.10-1.94 (m, 2H), 1.66 (dq, *J =* 17.6, 0.8 Hz, 3H), 1.06 (s, 9H).

¹⁹F-NMR (376 MHz, CDCl₃): δ -93.2 (dq, *J =* 21.8, 17.6 Hz).

ESI-MS *m*/*z:* 531.2 [M+Na]⁺.

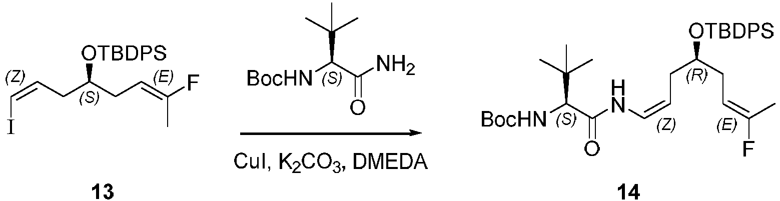

A flask charged with Boc-*tert*-Leu-CONH₂ (7.33 g, 31.9 mmol), Copper (I) iodide (1.21 g, 6.4 mmol), and Potassium carbonate (8.79 g, 63.7 mmol) was evacuated and filled with N₂ (x3). *N*,*N'-*Dimethylethylenediamine (1.35 mL, 12.7 mmol) and **13** (10.8 g, 21.2 mmol) in dry DMF (210 mL) were added at 23 °C. The flask was sealed, heated at 90 °C for 17 h and cooled to 23 °C. The reaction mixture was diluted with EtOAc (2 L) and H₂O (750 mL). The layers were separated and the organic layer was washed with H₂O (2 x 750 mL), dried over anhydrous Na₂SO₄, filtered and concentrated under vacuum. The residue was purified by flash chromatography on silica gel (Hexane:EtOAc from 100:1 to 6:1) to give **14** (7.56 g, 58% yield) as a colourless oil.

¹H NMR (400 MHz, CDCl₃): δ 7.77-7.63 (m, 4H), 7.51-7.32 (m, 6H), 7.06 (d, *J* = 10.5 Hz, 1H), 6.71-6.62 (m, 1H), 5.23 (d, *J* = 8.3 Hz, 1H), 4.92 (dt, *J* = 21.7, 7.9 Hz, 1H), 4.79-4.67 (m, 1H), 3.88-3.69 (m, 2H), 2.13-1.93 (m, 4H), 1.67 *(d, J=* 17.5 Hz, 3H), 1.43 (s, 9H), 1.07 (s, 9H), 0.97 (s, 9H).

ESI-MS *m*/*z:* 633.2 [M+Na]⁺.

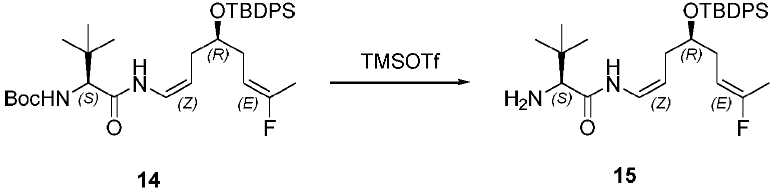

Trimethylsilyl trifluoromethanesulfonate (17.8 mL, 98.2 mmol) was added dropwise to a solution of **14** (7.5 g, 12.3 mmol) and 2,6-lutidine (14.3 mL, 123 mmol) in CH₂Cl₂ (310 mL) at 0 °C. The mixture was stirred for 5 min at 0 °C and for an additional hour at 23 °C. The reaction was quenched with an aqueous saturated aqueous solution of NH₄Cl (100 mL) and extracted with CH₂Cl₂. The layers were separated and the organic layer was washed with a solution 1 M NaOH (100 mL), an aqueous saturated solution of NaCl (100 mL) and H₂O (2 x 50 mL), dried over anhydrous NaSO₄, filtered and concentrated. The resulting oil was purified on silica gel (Hexane:EtOAc from 10:1 to 2:1) to provide **15** (7.5 g, 120% yield) as colourless oil contaminated with 2,6-lutidine.

¹H NMR (400 MHz, CDCl₃): δ 7.82-7.55 (m, 4H), 7.49-7.30 (m, 6H), 7.14 (m, 1H), 6.66 (m, 1H), 4.93 (dt, *J* = 21.9, 7.9 Hz, 1H), 4.78 (m, 1H), 3.79 (m, 1H), 2.25-2.12 (m, 3H), 2.06-1.98 (m, 2H), 1.64 (d, *J* = 17.7 Hz, 3H), 1.08-0.93 (m, 18H).

ESI-MS *m*/*z:* 511.3 [M+H]⁺.

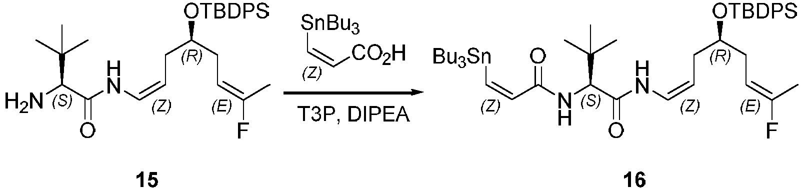

To a solution of **15** (6.3 g, 12.3 mmol) and (Z)-3-tributylstannylpropenoic acid (4.45 g, 12.3 mmol) in EtOAc (120 mL) was added DIPEA (5.37 mL, 30.8 mmol) and Propylphosphonic anhydride (T3P^{®}) (4.05 mL, 50% in EtOAc, 13.6 mmol) at 0 °C. The reaction mixture was stirred at 0 °C for 30 min, warmed to 23 °C and stirred for 3 h. The reaction mixture was quenched with H₂O (750 mL) and the layers were separated. The organic layer was washed with brine (750 mL), dried over anhydrous Na₂SO₄, filtered and concentrated. The residue was purified by flash chromatography (Hexane:EtOAc from 40:1 to 10:1) to give 16 (6.08 g, 58% yield) as colourless oil.

¹H NMR (400 MHz, CDCl₃): δ 7.77-7.69 (m, 4H), 7.55-7.37 (m, 6H), 7.13-7.06 (m, 1H), 7.07 (d*, J* = 12.3 Hz, 1H), 6.83 (d, *J* = 12.3 Hz, 1H), 6.75-6.63 (m, 1H), 6.51 (d, *J* = 9.4 Hz, 1H), 5.00 (dt, *J* = 21.4, 7.9 Hz, 1H), 4.85-4.72 (m, 1H), 4.42 (d, *J* = 9.6 Hz, 1H), 4.17 (q, *J* = 7.2 Hz, 1H), 3.83 (dt, *J* = 10.3, 5.4 Hz, 1H), 2.27-1.98 (m, 3H), 1.78-1.68 (m, 3H), 1.60-1.43 (m, 6H), 1.38-1.24 (m, 6H), 1.10 (s, 9H), 1.01 (s, 9H), 0.96-0-87 (m, 15H).

ESI-MS *m*/*z:* 877.3 [M+Na]⁺.

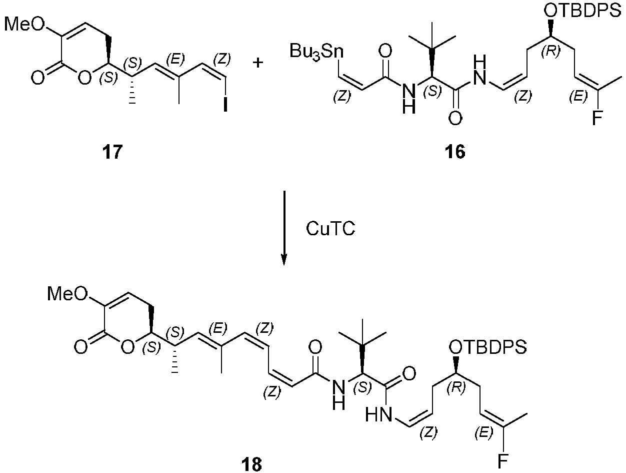

CuTC (870 mg, 4.57 mmol) was added in one portion to a solution of **16** (2.60 g, 3.04 mmol) and **17** (Comp 17a in WO2007144423) (1.06 g, 3.04 mmol) in *N*-Methyl-2-pyrrolidone (30 mL) at 0 °C. After stirring for 30 min at 0 °C and for 2 h at 23 °C, the reaction mixture was filtered through Al₂O₃ pad. The product was washed out using EtOAc (80 mL) and the solvent was evaporated. The residue was diluted with EtOAc (40 mL) and washed with 1.0 N HCl (3 x 20 mL). The combined organic layers were dried over anhydrous Na₂SO₄, filtered and evaporated under vacuum. The residue was purified by flash chromatography (Hexane:EtOAc from 2:1 to 1:1) to yield **18** (1.43 g, 58% yield) as white foam.

¹H NMR (400 MHz, CDCl₃): δ 7.71-7.62 (m, 4H), 7.51-7.35 (m, 6H), 7.31-7.21 (m, 1H), 7.12 (d, *J= 10.8* Hz, 1H), 6.90 (td, *J* = 11.5, 1.2 Hz, 1H), 6.65 (dd, *J* = 10.8, 9.1 Hz, 1H), 6.26-6.12 (m, 2H), 5.73-5.56 (m, 2H), 5.28 (d, *J* = 9.9 Hz, 1H), 4.94 (ddd, *J* = 21.5, 8.4, 7.2 Hz, 1H), 4.82-4.70 (m, 1H), 4.30 (d, *J =* 9.3 Hz, 1H), 4.21 (ddd, *J =* 10.5, 7.7, 5.1 Hz, 1H), 3.83-3.76 (m, 1H), 3.66 (s, 3H), 2.94-2.77 (m, 1H), 2.49-2.32 (m, 2H), 2.20-1.98 (m, 4H), 1.84 (s, 3H), 1.68 *(d, J=* 17.5 Hz, 3H), 1.16 (d, *J =* 6.7 Hz, 3H), 1.06 (s, 9H), 1.00 (s, 9H).

ESI-MS *m*/*z:* 785.3 [M+H]⁺, 807.3 [M+Na]⁺.

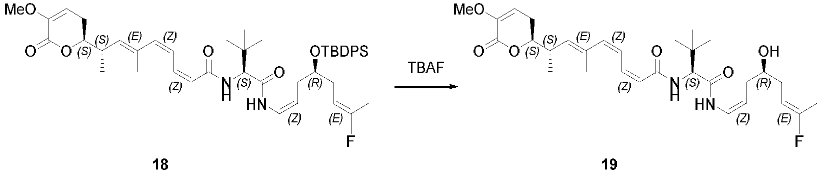

A solution of TBAF (3.57 mL, 1.0 M in THF, 3.57 mmol) was added dropwise to a solution of **18** (1.40 g, 1.78 mmol) in THF (27 mL) at 23 °C. After stirring for 2 h, the reaction was quenched with an aqueous saturated solution of NaHCO₃ (20 mL). The aqueous layer was extracted with EtOAc (3 x 20 mL), the combined organic layers were dried over anhydrous Na₂SO₄, filtered and evaporated. Flash chromatography (Hex:EtOAc from 2:1 to 1:1) of the residue provided **19** (680 mg, 70% yield) as a white foam.

¹H NMR (400 MHz, CD₃OD): δ 7.24 (td, *J =* 11.6, 1.2 Hz, 1H), 6.95 (td, *J =* 11.6, 1.2 Hz, 1H), 6.64 (d, *J* = 9.1 Hz, 1H), 6.29-6.14 (m, 1H), 5.92 (d, *J=* 11.4 Hz, 1H), 5.87 (t, *J* = 4.7 Hz, 1H), 5.34 (dt, *J* = 9.9, 1.5 Hz, 1H), 5.06 (dtd, *J=* 21.7, 8.0, 1.1 Hz, 1H), 4.92 (dt, *J =* 9.1, 7.5 Hz, 1H), 4.42 (s, 1H), 4.32 (dt, *J =* 8.1, 7.3 Hz, 1H), 3.64 (s, 3H), 3.66-3.59 (m, 1H), 2.95-2.86 (m, 1H), 2.55-2.43 (m, 2H), 2.41-2.02 (m, 4H), 1.93-1.78 (m, 6H), 1.15 (d, *J* = 6.6 Hz, 3H), 1.03 (s, 9H). ESI-MS *m*/*z:* 547.2 [M+H]⁺.

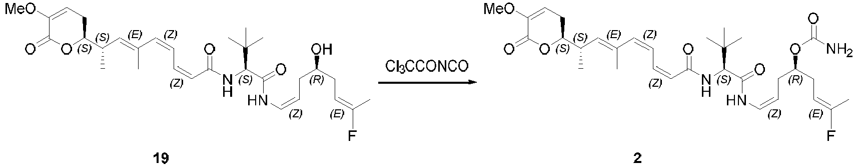

To a solution of **19** (670 mg, 1.23 mmol) in CH₂Cl₂ (70 mL) trichloroacetyl isocyanate (174 µL, 1.47 mmol) was added at 23 °C. The reaction was stirred at 23 °C for 30 min and then Al₂O₃ (9 g, previously activated with H₂O (15% w/w)) was added while stirring for 30 min. Then additional alumina Al₂O₃ added (4.5 g) and stirred for 45 min. The reaction mixture was filtered and washed out using a mixture of CH₂Cl₂:CH₃OH 10:1 and after evaporation of the filtrated at reduced pressure the product was purified by column chromatography (Hexane:EtOAc from 1: 1 to 1:2) to afford pure **2** (470 mg, 65% yield).

¹H NMR (400 MHz, CD₃OD): δ 7.24 (t, *J=* 11.6 Hz, 1H), 6.95 (td, *J =* 11.5, 1.1 Hz, 1H), 6.69 *(d, J=* 9.0 Hz, 1H), 6.19 *(d, J=* 11.6 Hz, 1H), 5.93 *(d, J=* 11.6 Hz, 1H), 5.87 (t, *J* = 4.7 Hz, 1H), 5.35 (d, *J =* 9.9 Hz, 1H), 5.02 (ddd, *J =* 21.2, 8.6, 7.5 Hz, 1H), 4.88-4.80 (m, 1H), 4.59 (m, 1H), 4.47 (s, 1H), 4.32 (m, 1H), 3.64 (s, 3H), 2.91 (dt, *J =* 9.8, 6.8 Hz, 1H), 2.58-2.41 (m, 3H), 2.42-2.13 (m, 3H), 1.91-1.86 (m, 6H), 1.15 (d, *J* = 6.7 Hz, 3H), 1.04 (s, 9H).

ESI-MS *m*/*z:* 590.2 [M+H]⁺.

### EXAMPLE 2 - SYNTHESIS OF 3

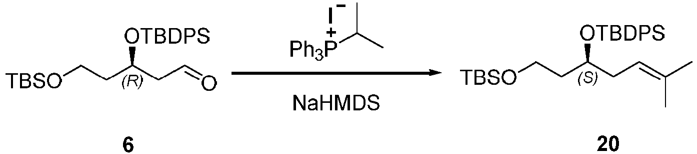

To a suspension of Isopropyltriphenylphosphonium iodide (60.6 g, 117 mmol) in THF (1 L) at - 78 °C was slowly added, via addition funnel, Sodium bis(trimethylsilyl)amide (140 mL, 1.0 M in THF, 140 mmol) over a period of 30 min at 0 °C. After stirring for 10 min, the reaction mixture was cooled at -78 °C and a solution of **6** (55 g, 117 mmol) in THF (500 mL) cooled at - 78 °C, was then added in 1 h. The reaction mixture was stirred at -78 °C for 1 h, warmed at 0 °C and stirred 30 min. Hexane (500 mL) was added and the resulting slurry was filtrated over silica and washing with more Hexane (1L). The filtrate was evaporated under reduced pressure to afford pure **20** (53.1 g, 83% yield).

¹H NMR (400 MHz, CDCl₃): δ 7.81-7.60 (m, 4H), 7.46-7.26 (m, 6H), 5.10-4.87 (m, 1H), 3.86 (m, 1H), 3.71-3.56 (m 2H), 2.10 (m, 2H), 1.69 (m, 2H), 1.60 (s, 3H), 1.38 (s, 3H), 1.04 (s, 9H), 0.84 (s, 9H), -0.02 (s, 6H).

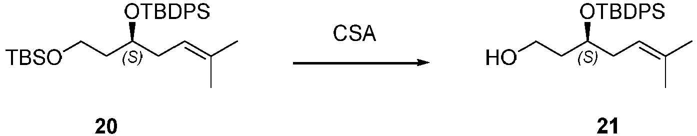

To a solution of **20** (53.37 g, 107 mmol) in CH₂Cl₂:CH₃OH (85:15, 2300 mL) Camphorsulfonic acid (7.48 g, 32 mmol) was added at 23 °C. The reaction mixture was stirred for 3 hours at 23 °C. A saturated aqueous solution of NaHCO₃ was added (pH= 9) and the layers were separated. The combined organic layer were dried over anhydrous Na₂SO₄, filtered, and the solvent was evaporated. The resulting oil was purified by column chromatography (Hex:EtOAc 100:1) to yield pure **21** (31.88 g, 78% yield).

¹H NMR (400 MHz, CDCl₃): δ 7.77-7.65 (m, 4H), 7.51-7.34 (m, 6H), 4.92-4.86 (m, 1H), 3.96-3.90 (m, 1H), 3.85-3.77 (m, 1H), 3.75-3.59 (m, 1H), 2.23-2.12 (m, 2H), 2.11-2.01 (m, 1H), 1.94-1.81 (m, 1H), 1.66-1.60 (m, 1H), 1.57 (s, 3H), 1.35 (s, 3H), 1.06 (s, 9H).

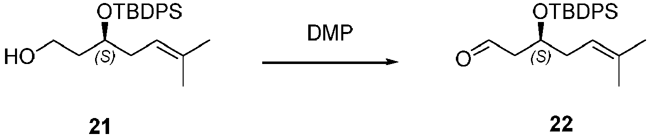

To a solution of **21** (25.3 g, 66.2 mmol) in anhydrous CH₂Cl₂ (660 mL) NaHCO₃ (16.7 g, 199 mmol) and Dess-Martin periodinane (42.11 g, 99 mmol) were successively added at 23 °C. The suspension was stirred for 1 hour and cooled at 0 °C. A mixture of an aqueous saturated solution of Na₂S₂O₃ and an aqueous saturated solution of NaHCO₃ (50:50, 350 mL) was carefully added and stirred an additional hour. The mixture was diluted with H₂O (100 mL) and the layers were separated. Aqueous phase was extracted with CH₂Cl₂ (100 mL) and the combined organic layers were dried over anhydrous Na₂SO₄, filtered and concentrated to dryness to afford **22** (24.6 g, 98%) which was used in the next step without further purification.

¹H NMR (400 MHz, CDCl₃): δ 9.71 (d, *J* = 2.8Hz, 1H), 7.77-7.57 (m, 4H), 7.54-7.31 (m, 6H), 5.05-4.90 (m, 1H), 4.21-4.19 (m, 1H), 2.49-2.43 (m, 2H), 2.25-2.15 (m, 2H), 1.61 (s, 3H), 1.40 (s, 3H), 1.05 (s, 9H).

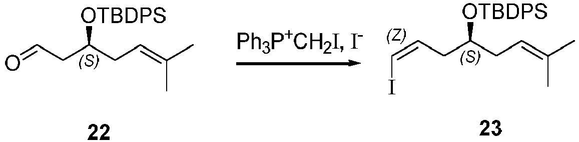

To a suspension of iodomethylenetriphenylphosphorane (24.6 g, 64.7 mmol) in anhydrous THF (670 mL) NaHMDS (90.6 mL, 1.0 M in THF, 90.6 mmol) was dropwise added over a period of 10 min at 23 °C. Then, the solution was cooled to -78 °C and precooled solution of **22** (24.6 g, 64.7 mmol) in anhydrous THF (100 mL) was dropwise added. The temperature was kept at -78 °C while the reaction mixture was stirred for 3 hours. Hexane (1 L) was added and the mixture filtered over Celite^{®} eluting with Hexane (1 L). Evaporation of volatiles gave an oil that was purified by flash chromatography (Hex:EtOAc 50:1) to afford **23** (21.71 g, 67% yield).

¹H NMR (400 MHz, CDCl₃): δ 7.76-7.60 (m, 4H), 7.51-7.31 (m, 6H), 6.36-6.14 (m, 2H), 5.09-4.93 (m, 1H), 3.95-3.80 (m, 1H), 2.36-2.24 (m, 2H), 2.13 (m, 2H), 1.63 (s, 3H), 1.43 (s, 3H), 1.06 (s, 9H).

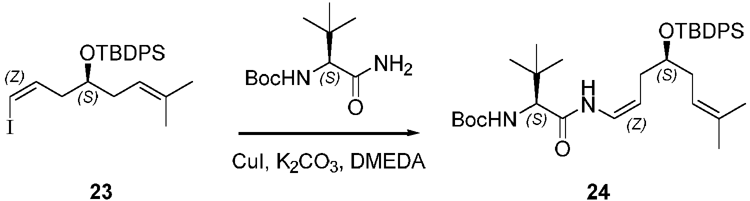

A flask charged with Boc-*tert*-Leu-CONH₂ (5.16 g, 22.4 mmol), Copper (I) iodide (850 mg, 4.5 mmol), and Potassium carbonate (6.20 g, 44.8 mmol) was evacuated and filled with N₂ (x3). *N*,*N'*-Dimethylethylenediamine (0.95 mL, 9.0 mmol) and **23** (7.54 g, 14.9 mmol) in dry DMF (150 mL) were added at 23 °C. The flask was sealed, heated at 90 °C for 17 h and cooled to 23 °C. The reaction mixture was diluted with EtOAc (2 L) and H₂O (500 mL). The layer were separated and the organic layer was washed with H₂O (3 x 500 mL), dried over anhydrous Na₂SO₄, filtered and concentrated under vacuum. The residue was purified by flash chromatography on silica gel (Hexane:EtOAc from 100:1 to 6:1) to give **24** (4.56 g, 52% yield).

¹H NMR (400 MHz, CDCl₃): δ 7.74-7.63 (m, 4H), 7.48-7.31 (m, 6H), 7.00 (d, *J* = 10.7 Hz, 1H), 6.66 (t, *J* = 9.9 Hz, 1H), 5.32-5.20 (m, 1H), 5.07-4.97 (m, 1H), 4.82 (m, 1H), 3.92-3.67 (m, 2H), 2.24-1.96 (m, 4H), 1.66 (s, 3H), 1.43 (s, 9H), 1.43 (s, 3H), 1.05 (s, 9H), 0.96 (s, 9H). ¹³C NMR (75 MHz, CDCl₃): δ 168.6, 156.1, 136.1, 135.0, 134.4, 134.1, 129.9, 127.8, 121.7, 120.4, 109.0, 73.0, 58.6, 35.5, 32.9, 28.5, 27.2, 26.7, 26.0, 19.5, 18.6, 18.0.

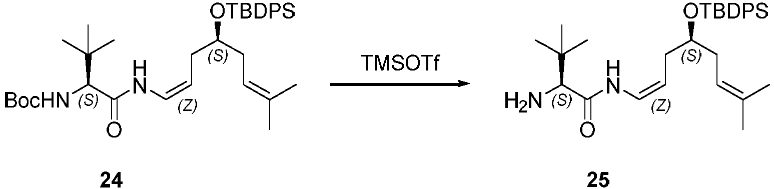

Trimethylsilyl trifluoromethanesulfonate (8.82 mL, 48.73 mmol) was added dropwise to a solution of **24** (4.55 g, 7.50 mmol) and 2,6-lutidine (6.11 mL, 52.48 mmol) in CH₂Cl₂ (75 mL) at 0 °C. The mixture was stirred for 5 min at 0 °C and for an additional hour at 23 °C. The reaction was quenched with an aqueous saturated aqueous solution of NH₄Cl (100 mL) and extracted with CH₂Cl₂. The layers were separated and the organic layer was washed with a solution 1 M NaOH (100 mL), an aqueous saturated aqueous solution of NaCl (100 mL) and H₂O (2 x 50 mL), dried over anhydrous NaSO₄, filtered and concentrated to provide crude 25 (4.2 g, 111% yield) as colourless oil contaminated with 2,6-lutidine which was used in the next without further purification.

¹H NMR (500 MHz, CDCl₃): δ 8.44 (d, *J=* 10.7 Hz, 1H), 7.70-7.62 (m, 4H), 7.44-7.31 (m, 6H), 6.65-6.57 (m, 1H), 5.00 (td, *J=* 6.6, 6.1, 1.2 Hz, 1H), 4.89 (m, 1H), 3.80-3.76 (m, 1H), 3.66-3.62 (m, 1H), 2.32-2.24 (m, 1H), 2.16-2.09 (m, 3H), 1.59 (s, 3H), 1.40 (s, 3H), 1.04 (s, 9H), 1.00 (s, 9H).

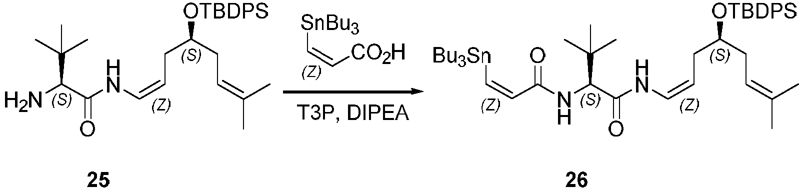

To a solution of 25 (1.19 g, 2.35 mmol) and (Z)-3-tributylstannylpropenoic acid (850 mg, 2.35 mmol) in EtOAc (23 mL) was added DIPEA (1.02 mL, 5.87 mmol) and Propylphosphonic anhydride (1.54 mL, 50% in EtOAc, 2.58 mmol) at 0 °C. The reaction mixture was stirred at 0 °C for 30 min, warmed to 23 °C and stirred for 1 h. The reaction mixture was quenched with H₂O (50 mL) and the layers were separated. The organic layer was washed with brine (50 mL), dried over anhydrous Na₂SO₄, filtered and concentrated. The residue was purified by flash chromatography (Hexane:EtOAc from 40:1 to 10:1) to give **26** (1.15 g, 75% yield) as colourless oil.

¹H NMR (400 MHz, CDCl₃): δ 7.74-7.65 (m, 4H), 7.55-7.31 (m, 6H), 7.02 (d, *J* = 12.2 Hz, 1H), 6.91 (d, *J* = 11.0 Hz, 1H), 6.75 (d, *J* = 12.3 Hz, 1H), 6.68-6.60 (m, 1H), 6.22 (d, *J* = 9.4 Hz, 1H), 5.03 (t, *J* = 7.2 Hz, 1H), 4.88-4.77 (m, 1H), 4.30 (d, *J =* 9.5 Hz, 1H), 3.85-3.75 (m, 1H), 2.19-1.95 (m, 4H), 1.67 (s, 3H), 1.62-1.35 (m, 6H), 1.46 (s, 3H), 1.38-1.18 (m, 6H), 1.05 (s, 9H), 0.95 (s, 9H), 0.94-0.70 (m, 15H).

ESI-MS *m*/*z:* 873.3 [M+Na]⁺.

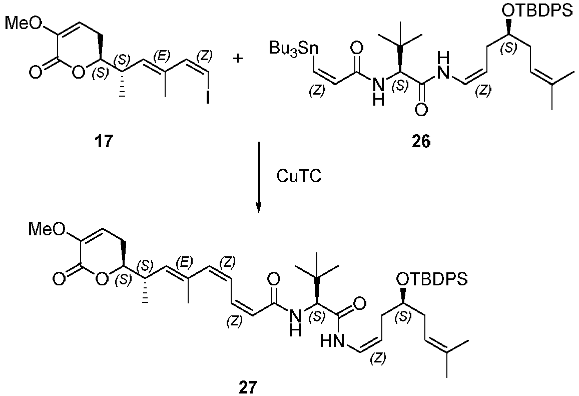

CuTC (720 mg, 3.79 mmol) was added in one portion to a solution of **26** (2.15 g, 2.53 mmol) and **17** (Comp. 17a in WO2007144423) (880 mg, 2.53 mmol) in *N*-Methyl-2-pyrrolidone (25 mL) at 0 °C. After stirring for 30 min at 0 °C and for 2 h at 23 °C, the reaction mixture was filtered through Al₂O₃ pad. The product was washed out using EtOAc (65 mL) and the solvent was evaporated. The residue was diluted with EtOAc (75 mL) and washed with 1.0 N HCl (3 x 20 mL). The combined organic layers were dried over anhydrous Na₂SO₄, filtered and evaporated under vacuum. The residue was purified by flash chromatography (Hexane:EtOAc from 2:1 to 1:1) to yield **27** (1.59 g, 80% yield) as white foam.

¹H NMR (400 MHz, CDCl₃): δ 7.74-7.62 (m, 4H), 7.50-7.33 (m, 6H), 7.33-7.20 (m, 1H), 7.13 (d, *J* = 10.7 Hz, 1H), 6.88 (td, *J* = 11.5, 1.2 Hz, 1H), 6.63 (t, *J* = 9.8 Hz, 1H), 6.27 (d, *J* = 9.2 Hz, 1H), 6.16 (d, *J* = 11.8 Hz, 1H), 5.73-5.57 (m, 2H), 5.27 (d, *J* = 9.8 Hz, 1H), 5.01 (t, *J* = 7.1 Hz, 1H), 4.95-4.75 (m, 1H), 4.34-4.25 (m, 1H), 4.25-4.15 (m, 1H), 3.79 (t, *J =* 5.8 Hz, 1H), 3.65 (s, 3H), 2.91-2.76 (m, 1H), 2.48-2.34 (m, 2H), 2.25-2.08 (m, 4H), 1.83 (d, *J =* 1.4 Hz, 3H), 1.65 (s, 3H), 1.43 (s, 3H), 1.15 (d, *J =* 6.6 Hz, 3H), 1.04 (d, *J =* 1.4 Hz, 9H), 0.98 (s, 9H).

ESI-MS *m*/*z:* 781.4 [M+H]⁺.

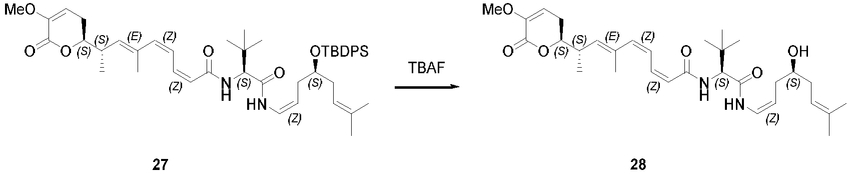

A solution of TBAF (4.07 mL, 1.0 M in THF, 4.07 mmol) was added dropwise to a solution of **27** (1.59 g, 2.04 mmol) in THF (31 mL) at 23 °C. After stirring for 2 h, the reaction was quenched with an aqueous saturated solution of NaHCO₃ (45 mL). The aqueous layer was extracted with EtOAc (3 x 20 mL), the combined organic layers were dried over anhydrous Na₂SO₄, filtered and evaporated. Flash chromatography (Hex:EtOAc from 2:1 to 1:1) of the residue provided **28** (806 mg, 73% yield) as a white foam.

¹H NMR (400 MHz, CD₃OD): δ 7.24 (t, *J =* 11.5 Hz, 1H), 6.95 (t, *J* = 11.6 Hz, 1H), 6.62 (d, *J* = 9.0 Hz, 1H), 6.18 (d, *J=* 11.6 Hz, 1H), 5.92 (d, *J =* 11.6 Hz, 1H), 5.86 (t, *J =* 4.7 Hz, 1H), 5.34 (d, *J* = 9.0 Hz, 1H), 5.20 *(d, J=* 7.1 Hz, 1H), 4.99-4.89 (m, 1H), 4.41 (s, 1H), 4.37-4.23 (m, 1H), 3.63 (s, 3H), 3.65-3.59 (m, 1H), 2.94-2.86 (m, 1H), 2.46 (m, 2H), 2.39-2.26 (m, 2H), 2.26-2.12 (m, 2H), 1.86 (s, 3H), 1.70 (s, 3H), 1.61 (s, 3H), 1.14 (d, *J =* 6.6 Hz, 3H), 1.02 (s, 9H).

ESI-MS *m*/*z:* 543.3 [M+H]⁺.

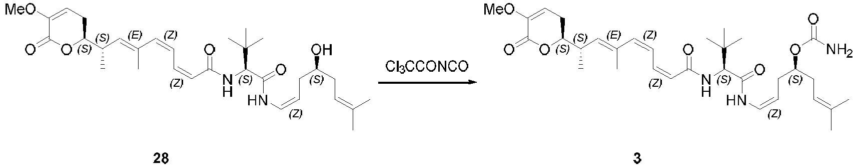

To a solution of **28** (600 mg, 1.11 mmol) in CH₂Cl₂ (66 mL) trichloroacetyl isocyanate (157 µL, 1.32 mmol) was added at 23 °C. The reaction was stirred at 23 °C for 30 min and then Al₂O₃ (9 g, previously activated with H₂O (15% w/w)) was added while stirring for 30 min. Then additional alumina Al₂O₃ added (4.5 g) and stirred for 45 min. The reaction mixture was filtered and washed out using a mixture of CH₂Cl₂:CH₃OH 10:1 and after evaporation of the filtrated at reduced pressure the product was purified by column chromatography (Hexane:EtOAc from 1: 1 to 1:2) to afford pure 3 (590 mg, 91% yield).

¹H NMR (400 MHz, CD₃OD: δ 8.70 (d, *J =* 10.5 Hz, 1H), 7.33 (d, *J=* 11.6 Hz, 1H), 6.90 (t, *J =* 11.5 Hz, 1H), 6.81 (t, *J=* 9.7 Hz, 1H), 6.52 (d, *J=* 9.3 Hz, 1H), 6.16 (d, *J=* 11.5 Hz, 1H), 5.71 (d, *J* = 11.6 Hz, 1H), 5.63 (d, *J =* 6.6 Hz, 1H), 5.37-5.04 (m, 4H), 4.93-4.73 (m, 1H), 4.53-4.34 (m, 2H), 4.34-4.19 (m, 1H), 3.66 (s, 3H), 2.93-2.77 (m, 1H), 2.57-2.34 (m, 3H), 2.34-2.25 (m, 2H), 2.22-2.06 (m, 1H), 1.82 (s, 3H), 1.62 (s, 3H), 1.59 (s, 3H), 1.15 (d, *J* = 6.5 Hz, 3H), 1.04 (s, 9H).

ESI-MS *m*/*z:* 586.4 [M+H]⁺.

As required, further guidance on general synthesis relating to compounds 2 and 3 can be gained from WO 2007/144423.

### EXAMPLE 3 IN VITRO BIOASSAYS FOR THE DETECTION OF ANTITUMOR ACTIVITY

The aim of this assay is to evaluate the *in vitro* cytostatic (ability to delay or arrest tumor cell growth) or cytotoxic (ability to kill tumor cells) activity of the samples being tested.

### Cell Lines

| **Name** | **N° ATCC (when applicable)** | **Species** | **Tissue** | **Characteristics** |
|---|---|---|---|---|
| A549 | CCL-185 | human | lung | lung carcinoma (NSCLC) |
| HT29 | HTB-38 | human | colon | colorectal adenocarcinoma |
| MDA-MB-231 | HTB-26 | human | breast | breast adenocarcinoma |
| PSN-1 | Ref. 1 | human | pancreas | pancreatic adenocarcinoma |

Ref. 1 Yamada, T. et al. (1986) Establishment of a human pancreatic adenocarcinoma cell line (PSN-1) with amplifications of both c-myc and activated c-Ki-ras by a point mutation. Biochem. Biophys. Res. Commun. 140, 167-173.

### EVALUATION OF CYTOTOXIC ACTIVITY USING THE SRB COLORIMETRIC ASSAY

A colorimetric assay, using sulforhodamine B (SRB) reaction has been adapted to provide a quantitative measurement of cell growth and viability (using the technique developed by Skehan et al., J. Natl. Cancer Inst. 1990 and following the protocol detailed in V. Vichai and K. Kirtikara (2006) Nature Protoc. 1, 1112-1116.)

This form of assay employs 96-well cell culture microplates. All the cell lines used in this study were obtained from the American Type Culture Collection (ATCC), unless otherwise indicated, and derive from different types of human cancer.

Cells were maintained in Dulbecco's Modified Eagle Medium (DMEM) (for A549, HT-29 and MDA-MB-231) or RPMI (for PSN-1) supplemented with 10% Fetal Bovine Serum (FBS), 2mM L-glutamine, 100 U/mL penicillin and 100 U/mL streptomycin at 37 °C, 5% CO₂ and 98% humidity. For the experiments, cells were harvested from subconfluent cultures using trypsinization and resuspended in fresh medium before counting and plating.

Cells were seeded in 96 well microtiter plates, at 5 x 10³ cells per well in aliquots of 150 µL, and allowed to attach to the plate surface for 18 hours (overnight) in drug free medium. After that, one control (untreated) plate of each cell line was fixed (as described below) and used for time zero reference value. Culture plates were then treated with test compounds (50 µL aliquots of 4X concentrated compound stock solutions made in complete culture medium) using ten serial dilutions (concentrations ranging from 10 to 0.00262 µg/mL) and triplicate cultures (final concentration of DMSO being 1%). After 72 hours treatment, the antitumor effect was measured by using the SRB methodology: Briefly, cells were washed twice with PBS, fixed for 15 min in 1% glutaraldehyde solution at room temperature, rinsed twice in PBS, and stained in 0.4% SRB solution for 30 min at room temperature. Cells were then rinsed several times with 1% acetic acid solution and air-dried at room temperature. SRB was then extracted in 10 mM trizma base solution and the absorbance measured in an automated spectrophotometric plate reader at 490 nm. Effects on cell growth and survival were estimated by applying the NCI algorithm (Boyd MR and Paull KD. Drug Dev. Res. 1995, 34, 91-104).

Using the mean ± SD of triplicates, a dose-response curve was automatically generated using nonlinear regression analysis to a 4-parameter logistic curve. Three reference parameters were calculated (NCI algorithm) by automatic interpolation: GI₅₀ = compound concentration that produces 50% cell growth inhibition, as compared to control cultures.

Table 1 illustrates data on the biological activity (GI₅₀) of compounds of the present invention (GI₅₀ value).

**Table 1. Cytotoxicity assay-Activity Data (GI₅₀ Molar)**

| **Compounds** | | **GI₅₀ (M)** | | | |
|---|---|---|---|---|---|
| | | **A549** | **HT29** | **MDA-MB-231** | **PSN-1** |
| 2 | 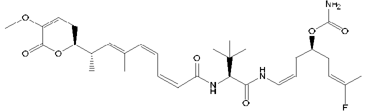 | 4.55E-11 | 1.31E-11 | 4.87E-11 | 2.27E-11 |
| 3 | 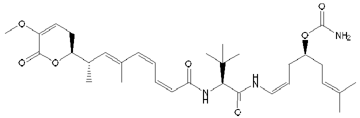 | 5.98E-11 | 2.22E-11 | 9.05E-11 | 1.43E-10 |
| PM060184 | 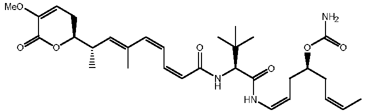 | 1.71E-10 | 8.30E-11 | 2.07E-10 | 1.87E-10 |
| PM050489 | 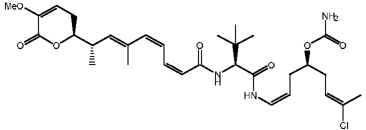 | 8.59E-11 | 3.91E-11 | 1.37E-10 | 1.90E-10 |

It can be seen that the compounds of the present invention have surprisingly improved activity over the comparative compounds in all cell lines. The compounds of the present invention are shown to have high activity *in vitro,* in particular low nanomolar activity across a range of different cell lines. This demonstrates that the compounds according to the present invention exhibit high cytoxiticy towards cancer cells and are useful in the treatment of cancer. Importantly, the compounds of the present invention show improved activity when compared against the reference compounds. In particular, compound 2 is between 3.8 to 8.2 times more active than reference compound PM060184. In addition, compound 3 is between 1.3 to 3.7 times more active than reference compound PM060184. In addition, compound 2 is between 1.9 to 7.9 times more active than reference compound PM050489. In addition, compound 3 is between 1.3 to 1.8 times more active than reference compound PM050489.

### Example 4. MTD and MTMD determination

### Example 4.1. MTD and MTMD determination in female athymic Nude-Fox1 nu/nu mice.

Female Athymic Nude-Fox1 nu/nu mice (Envigo) were utilized for all experiments. Animals (N=10/cage) were housed in individually ventilated cages (Sealsafe Plus^{®}, Techniplast S.P.A.), on a 12-hour light-dark cycle at 21-23 °C and 40-60% humidity. Mice were allowed free access to irradiated standard rodent diet (Tecklad 2914C) and sterilized water. Animals were acclimated for five days prior to being individually tattoo-identified. Animal protocols were reviewed and approved according to the regional Institutional Animal Care and Use Committees.

Mice were randomly allocated into experimental groups and intravenously administered, once for the MTD (Maximum Tolerated Dose) determination or one administration a week during three consecutive weeks, for the MTMD (Maximum Tolerated Multiple Dose) determination study. The animals were administered with white formulation or with compound dissolved in the experimental formulation at different concentrations. The volume administered was always 10 mL/kg. Once administered, animals were monitored for clinical signs of systemic toxicity, changes in body weight and mortality up to 14 days after the administration.

MTD results are summarized in **Table 2**

**Table 2**

| **Compound** | **Route / Schedule** | **MTD (mg/kg)** |
|---|---|---|
| PM060184 | iv / SD | 16.0 |
| PM050489 | | 0.075 |
| **3** | | 1.0 |
| **2** | | 2.0 |

| | | |
|---|---|---|
| iv, intravenously SD, single dose | | |

MTMD results are summarized in **Table 3**

**Table 3**

| **Compound** | **Route / Schedule** | **MTMD (mg/kg)** |
|---|---|---|
| PM060184 | iv / Q7dx3 | 16.0 |
| PM050489 | | 0.075 |
| **3** | | 1.0 |
| **2** | | 2.0 |

| | | |
|---|---|---|
| iv, intravenously Q7dx3, three cumulated doses administered in a weekly basis | | |

### Comparative Example. In vivo xenograft

### CELL LINES

| **Name** | **N° ATCC** | **N° ECCC*** | **Species** | **Tissue** | **Characteristics** |
|---|---|---|---|---|---|
| H460 | HTB-177 | - | human | lung, pleural effusion | NSCLC |

| | | | | | |
|---|---|---|---|---|---|
| * European Collection of Cell Cultures | | | | | |

H460 cells were maintained in vitro at 37 °C with 5% CO2 in Dulbecco's Modified Eagle's Medium (Sigma-Aldrich, Co). Culture cells were passaged every 3 to 5 days upon reaching confluence. Each animal was subcutaneously implanted (on the right flank using 26G needle and a 1 cc syringe) at 4-6 weeks of age with 5x10⁶ H460 cells suspended in 0.05 mL of a solution consisting of 50% Matrigel (Corning Incorporated Life Sciences) and 50% medium without serum or antibiotics.

Antitumor activity of PM050489 was evaluated in one human tumor model, NSCLC (H460) xenografted in immunodepressed mice. All treatments were intravenously administered at their MTD.

When tumors reached *ca.* 150-200 mm³, tumor bearing animals *(N =* 10/group) were randomly allocated into the following treatment groups:
PM050489 (0.08 mg/kg)
Placebo

Treatments were intravenously administered once per week for 2 consecutive weeks (days 0 and 7).

Tumor dimensions and body weights were recorded 3 times per week starting from the first day of treatment (Day 0). Treatments producing >20% lethality and/or 20% net body weight loss were considered toxic. Tumor volume was calculated using the equation (a·b²)/2, where *a* and *b* were the longest and shortest diameters, respectively. Animals were euthanized when their tumors reached *ca.* 2,000 mm³ and/or severe necrosis was seen. Median, mean and SD were calculated for tumor volume on each measurement day.

Antitumor effect was calculated by using ΔT/ΔC (%) and tumor growth delay (TGD). ΔT/ΔC was defined as a percentage of the change in tumor volume for each treated (*T*) and placebo (*C*) group. ΔT/ΔC was calculated inmediately before the 2^{nd} dose and on the 4^{th} week of the study. TGD was expressed as a percentage by which the treated group volume was delayed and calculated by applying [(Dₜ-D_{c})/D_{c}]·100, where *Dₜ* and *D_{c}* were the median times in days for treated and control groups in attaining a specified volume, fixed as 1,000 mm³.

Treatment tolerability was assessed by monitoring body weight evolution, clinical signs as well as evidences of local damage in the injection site.

All placebo-treated animals died or were sacrificed for ethical reasons from days 0 to 10. Tumors in this group had a doubling time calculated as 2.6 days (95 % CI, 2.2 to 3.2).

PM050489 treatment (0.08 mg/kg) was prematurely finished because of severe necrosis in the tumor masses and/or decreases in body weight values. The antitumor effect parameters are shown in **Table 7.**

**Table 7. Antitumor effect parameters in mice bearing H460 xenografts.**

| **Compound** | **Dose (mg/kg)** | **ΔT/ΔC (%)** | | **TGD (%)** |
|---|---|---|---|---|
| | | **Day 7** | **Day 24** | |
| Placebo | 0 | - | - | |
| PM050489 | 0.08 | 103.5 | NC | 40.0 |

| | | | | |
|---|---|---|---|---|
| ΔT/ΔC = (T₀-T_{D}/C₀-C_{D}) · 100. TGD, tumor growth delay (target volume 1,000 mm³). NC, not calculated: 100 % of reference animals (placebo or treated) died or were sacrificed before the evaluation time point. | | | | |

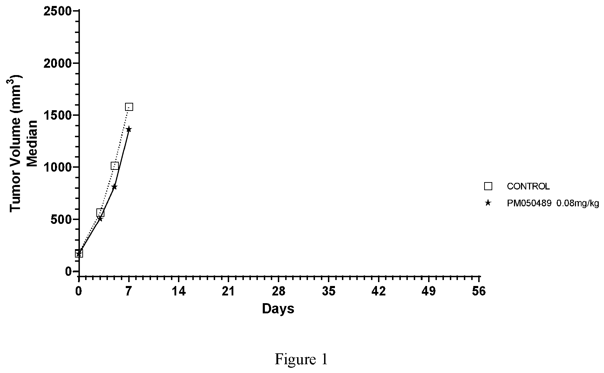

Tumor growth curves are displayed in Figure 1.

### Examples 5 - 7. In vivo xenografts

Female athymic nu/nu mice (Harlan Laboratories Models, S.L. Barcelona, Spain or Envigo, Spain) were utilized for all experiments. Animal were housed in individually ventilated cages Sealsafe^{®} Plus, Techniplast S.P.A.), up to ten per cage on a 12-hour light-dark cycle at 21-23 °C and 40-60 % humidity. Mice were allowed free access to irradiated standard rodent diet (Tecklad 2914C) and sterilized water. Animals were acclimated for at least 5 days prior to tumor implantation with a tumor cell suspension.

### CELL LINES

| **Name** | **N° ATCC** | **N° ECCC*** | **Species** | **Tissue** | **Characteristics** |
|---|---|---|---|---|---|
| H460 | HTB-177 | - | human | lung, pleural effusion | NSCLC |
| HGC27 | - | 94042256 | human | gastric | Gastric carcinoma |
| HCT116 | CCL-247 | - | human | colon | Colorectal carcinoma |

| | | | | | |
|---|---|---|---|---|---|
| * European Collection of Cell Cultures | | | | | |

H460 cells were maintained *in vitro* at 37 °C with 5% CO₂ in Dulbecco's Modified Eagle's Medium (Sigma-Aldrich, Co). Culture cells were passaged every 3 to 5 days upon reaching confluence. Each animal was subcutaneously implanted (on the right flank using 26G needle and a 1 cc syringe) at 4-6 weeks of age with 5x10⁶ H460 cells suspended in 0.05 mL of a solution consisting of 50% Matrigel^{®} (Corning Incorporated Life Sciences) and 50% medium without serum or antibiotics.

HGC27 cells were maintained *in vitro* at 37 °C with 5% CO₂ in Iscove's Modified Dulbecco's Medium (Sigma Aldrich, Co). Culture cells were passage every 3 to 5 days on reaching confluence. Each animal was subcutaneously implanted (on the right flank using 26G needle and a 1 cc syringe) at 4-6 weeks of age with 5x10⁶ HGC-27 cells suspended in 0.05 mL of a solution consisting of 50% Matrigel^{®} (Corning Incorporated Life Sciences), 50% medium without serum or antibiotics.

HCT116 cells were maintained in vitro at 37°C, 5% Co₂ in McCoy's 5A Medium (Sigma Aldrich, Co.). Culture cells were passage every 3 to 5 days on reaching confluence. Each animal was subcutaneously implanted (on the right flank using 26G needle and a 1 cc syringe) at 4-6 weeks of age with 5x10⁶ HCT116 cells suspended in 0.05 mL of a solution consisting of 50% Matrigel^{®} (Corning Incorporated Life Sciences) and 50% medium without serum or antibiotics.

Antitumor activity of compounds **2** and **3** was compared to PM060184 in three different human tumor models, in which PM060184 has been demostrated to be highly active: NSCLC (H460), gastric (HGC-27), and colon (HCT-116) xenografted in immunodepressed mice. All treatments were intravenously administered on a weekly basis at their MTD, ¼ MTD and 1/16 MTD (high, medium and low dose).

Briefly:
- 4 to 6 week-old athymic nu/nu female mice were subcutaneously implanted with tumor cells growing in culture.
- When tumors reached a certain volume, tumor bearing animals (N = 8-10/group) were randomly allocated into treatment groups.
- Tumor dimensions and body weights were recorded 2-3 times per week starting from the first day of treatment (Day 0). Treatments producing >20% lethality and/or 20% net body weight loss were considered toxic. Tumor volume was calculated using the equation (a·b²)/2, where a and b were the longest and shortest diameters, respectively. Animals were euthanized when their tumors reached ca. 2,000 mm³ and/or severe necrosis was seen.
- Median was calculated for tumor volume on each measurement day.
- Antitumor effect was assessed comparing tumor volume data from groups following the 1st, 2nd, 3rd study weeks, or more depending on the case, using a two-tailed Mann-Whitney U test. The data are presented as medians and interquartile range (IQR).
- Complete tumor regression (CR) was defined when tumor volume < 63 mm³ for 2 or more consecutive measurements.
- Treatment tolerability was assessed by monitoring body weight evolution, clinical signs of systemic toxicity as well as evidences of local damage at the injection site.

PM060184 was provided in the form of freeze-dried vials of lyophilized product. Each vial was reconstituted with water for infusion to a concentration of 2.5 mg/mL.

Compounds **2** and **3** were provided in the powder form and were solubilized with hydroxy-propyl-β-cyclodextrin (HPβCD), reaching a concentration of 1 mg/ml or 0.2 mg/ml, respectively.

Placebo was provided in the form of lyophilised cake containing 1.2g of hydroxy-propyl-β-cyclodextrin (HPβCD) which was reconstituted with water for infusion.

In these experiments, compounds **2, 3** and PM060184, as well as placebo, were intravenously administered once per week for 3 consecutive weeks, on Days 0, 7 and 14, whenever it was possible.

**Example 5.** *In vivo* studies to determine the effect of compounds **2** and **3** in a NSCLC xenograft model, H460

The experiment was carried out as previously explained. When tumors reached ca. 160-170 mm³, tumor bearing animals (N =10/group) were randomly allocated into the following treatment groups:

| **Group** | **Group number** | **Dose (mg/kg)** | **Schedule** |
|---|---|---|---|
| Placebo | G01 | 0.0 | |
| PM060184 | G02 | 16.0 | |
| | G03 | 4.0 | |
| | G04 | 1.0 | |
| Comp. **2** | G05 | 2.0 | Q7dx3, IV |
| | G06 | 0.5 | |
| | G07 | 0.125 | |
| Comp. **3** | G08 | 1.0 | |
| | G09 | 0.25 | |
| | G10 | 0.0625 | |

Treatments were initiated on Day 0.

Animals in the placebo group were sacrificed due to tumor volume (> 2,000 mm³) and/or tumor necrosis between days 9 and 12. In this experiment, H460 tumors had a doubling time of 2.7 days.

Treatment with compounds **2** and **3** caused, in tumor bearing animals, a high but reversible mean body weight reduction. Compound **2** (2.0 mg/kg) treatment caused a mean body weight reduction of (ca. -13.4%) recorded on Day 9 and compound **3** (1.0 mg/kg) (ca. -18.5%) recorded on Day 9. When the animals were treated with PM060184 at 16 mg/kg a moderate body weight decrease was recorded on Day 12 (ca. -6.8%). All the treated animals returned back to normal when the treatments stopped. No other clinical signs of systemic toxicity were seen.

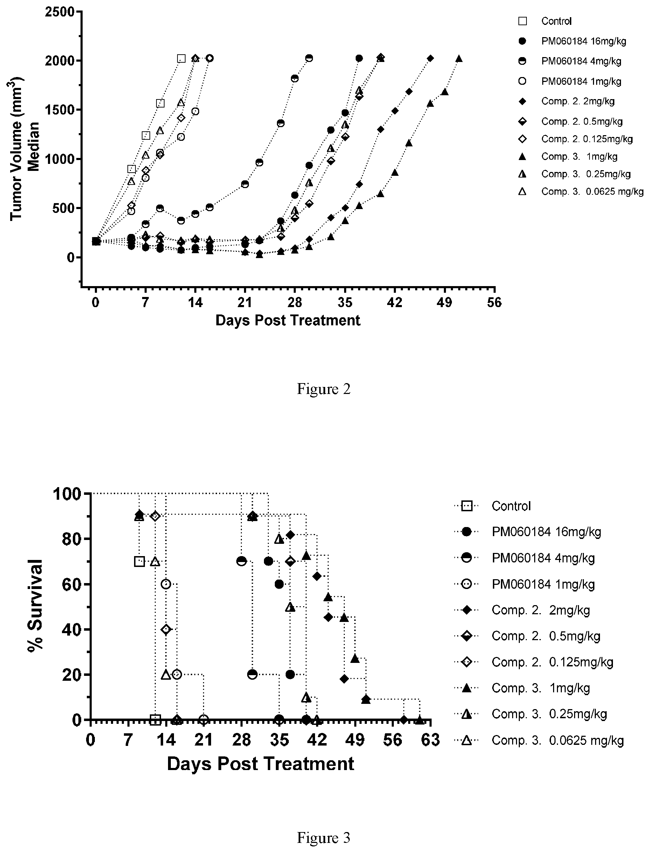

Tumor growth curves are displayed in Figure 2. The administered treatments showed a dose-dependent antitumor activity in animals bearing H460 tumor xenografts. Treatments administered at high or medium dose showed a very strong or strong, respectively, antitumor activity whereas the low dose resulted in limited or no antitumor activity in this model. A 100% of animals treated with the highest dose of compound **2** (2.0 mg/kg) or compound **3** (1.0 mg/kg) had complete tumor remissions during the experiment. The mean time frame during which the animals had tumor remission was 8.8 days for animals treated with compound **2** (at 2.0 mg/kg), and 7.8 days for animals treated with compound **3** (at 1.0 mg/kg). Tumor remissions lasted more than 48 days and 30.9 days for the groups treated with compound **2** at 2.0 mg/kg and 0.5 mg/kg, respectively; tumor remissions lasted more than 45.1 days for the group treated with compound **3** at 1.0 mg/kg. Four out of 10 animals treated with PM060184 at 16 mg/kg experienced complete tumor remission which lasted two days.

Placebo-treated group had a median (IQR) tumor volume of 1241 (1025-1506) mm³ on day 7, respectively. On days 7, 14, 21, 28 and 35, PM160184-treated animals had a median (IQR) tumor volume of 100.1 (80.6-119.0), 97.8 (74.5-149.3), 129.4 (83.5-172.3), 631.0 (454.0-873.9) and 1470 (1470-1666) mm³, respectively. On the same days, compound **2**-treated animals had a median (IQR) tumor volume of 114.7 (97.2-131.6), 80.9 (61.1-109.3), 53.5 (33.4-59.7), 95.0 (73.2-119.1) and 505.4 (428.9-583.7) mm³, respectively. On days 7, 14, 21, 28 and 35, compound **3**-treated animals had a median (IQR) tumor volume of 123.9 (93.4-157.6), 80.3 (71.0-83.5), 59.3 (38.7-62.5), 77.6 (55.3-85.1) and 374.8 (257.4-438.2) mm³, respectively. Compared to placebo, PM060184, compound 2 and compound **3**-treated animals, administered at their higher doses (16, 2 or 1 mg/kg, respectively) experienced a very strong, statistically significant tumor reduction on Day 7 which was the last measurement day in euthanized, placebo-treated group. Compared to PM060184 (16 mg/kg), compound **2** at 2.0 mg/kg resulted in a stronger, statistically significant, antitumor activity in this model, as well as compound **3** at 1.0 mg/kg. When compounds **3** and **2** were compared after their administration at the corresponding MTD's (1.0 mg/kg or 2.0 mg/kg, respectively), the antitumor activity of compound **3** resulted in statistically significant higher antitumor activity than compound **2** in this model **(Table 8).**

**Table 8. Tumor volumes (TV) obtained in mice bearing H460 xenografts and treated with PM060184, 2 and 3.**

| **Compound** | **Day** | **TV, mm3 median (IQR)** | ***P*** | ***P*** | ***P*** |
|---|---|---|---|---|---|
| G01 Placebo | 7 | 1241 (1025-1506) | | | |
| G02 PM060184, 16 mg/kg | 7 | 100.1 (80.6-119.0) | <0.0001^{a} | | |
| | 14 | 97.8 (74.5-149.3) | -^{b} | | |
| | 21 | 129.4 (83.5-172.3) | -^{b} | | |
| | 28 | 631.0 (454.0-873.9) | -^{b} | | |
| | 35 | 1470 (1470-1666) | -^{b} | | |
| G05 Comp. **2**, 2 mg/kg | 7 | 114.7 (97.2-131.6) | 0.0002^{a} | -^{c} | |
| | 14 | 80.9 (61.1-109.3) | -^{b} | -^{c} | |
| | 21 | 53.5 (33.4-59.7) | -^{b} | 0.0002^{c} | |
| | 28 | 95.0 (73.2-119.1) | -^{b} | <0.0001^{c} | |
| | 35 | 505.4 (428.9-583.7) | -^{b} | 0.0007^{c} | |
| G06 Comp **3,** 1mg/kg | 7 | 123.9 (93.4-157.6) | <0.0001^{a} | -^{d} | |
| | 14 | 80.3 (71.0-83.5) | -^{b} | -^{d} | |
| | 21 | 59.3 (38.7-62.5) | -^{b} | 0.0002^{d} | |
| | 28 | 77.6 (55.3-85.1) | -^{b} | <0.0001^{d} | |
| | 35 | 374.8 (257.4-438.2) | -^{b} | 0.0007^{d} | 0.0355^{e} |
| Data are presented as medians and interquartile range (IQR) | | | | | |
| ^{a} P value for Mann-Whitney *U* test respect to placebo treated. | | | | | |
| ^{b} No placebo group to compare with | | | | | |
| ^{c} *P* value for Mann-Whitney *U* test, comparison PM060184-treated group respect to comp. **2**-treated group. | | | | | |
| ^{d} *P* value for Mann-Whitney *U* test, comparison PM060184-treated group respect to comp. **3**-treated group. | | | | | |
| ^{e} P value for Mann-Whitney *U* test, comparison comp **2**-treated group respect to comp. **3**-treated group. | | | | | |

**Table 9. Survival and statistic result for mice bearing H460 xenografted tumors and treated with PM060184, 2 and 3.**

| **Group** | **Treatment** | **Dose (mg/kg)** | **Median survival time (days)** | ***P*** | ***P*** | ***P*** |
|---|---|---|---|---|---|---|
| 01 | Control | 0.0 | 12 | | | |
| 02 | PM060184 | 16.0 | 37 | <0.0001^{a} | | |
| 03 | | 4.0 | 30 | <0.0001^{a} | | |
| 04 | | 1.0 | 16 | <0.0001^{a} | | |
| 05 | Comp. 2 | 2.0 | 45.5 | <0.0001^{a} | 0.0002^{b} | |
| 06 | | 0.5 | 40 | <0.0001^{a} | <0.0001^{b} | |
| 07 | | 0.125 | 14 | <0.0001^{a} | -^{b} | |
| 08 | Comp. **3** | 1.0 | 48 | <0.0001^{a} | <0.0001^{c} | -^{d} |
| 09 | | 0.25 | 38.5 | <0.0001^{a} | 0.0001^{c} | -^{d} |
| 10 | | 0.0625 | 14 | 0.0049^{a} | <0.0219^{c} | -^{d} |
| ^{a} *P* value for log rank test , treated groups compared to placebo group. | | | | | | |
| ^{b} *P* value for log rank test, comparison PM060184-treated group respect to comp. **2**-treated group. | | | | | | |
| ^{c} *P* value for log rank test, comparison PM060184-treated group respect to comp. **3**-treated group. | | | | | | |
| ^{d} *P* value for log rank test, comparison comp **2**-treated group respect to comp. **3**-treated group. | | | | | | |

Kaplan-Meier survival curves are shown in Figure 3.

PM060184 treatment, statistically significantly (p<0.0001) increased the survival time compared to the placebo-treated group at any dose: placebo-treated median survival time 12 days vs. 37, 30 or 16 days, respectively).

Compound **2** treatment, statistically significantly (p<0.0001) increased the survival time compared to the placebo-treated group at all the doses assayed: placebo-treated median survival time 12 vs. 45.5, 40 or 14 days, respectively. At the high and medium doses, compound 2 treatments resulted in a statistically significant increase in the survival time compared to PM060184-treated group (p=0.0002 and P<0.0001).

Compound **3** treatment, statistically significantly increased the survival time compared to placebo-treated group at 1.0 mg/kg (high dose), 0.25 mg/kg (medium dose) and 0.0625 mg/kg (low dose): placebo-treated median survival time 12 vs. 45.5, 40 or 14 days (p<0.0001, p<0.0001 and p=0.0049, respectively). Comparing compound **3** and PM060184 treatments, at high, medium or low dose, the compound **3** treated group resulted in a statistically significant increase in the survival time compared to PM060184-treated group (p<0.0001, p=0.0001 and p=0.0219) at high, medium and low dose, respectively.

### Example 6. In vivo studies to determine the effect of the compounds 2 and 3 in a gastric xenograft model, HGC-27

The experiment was carried out as previously explained. When tumors reached ca. 165-175 mm³, tumor bearing animals (N =8/group) were randomly allocated into the following treatment groups:

| **Group** | **Group number** | **Dose (mg/kg)** | **Schedule** |
|---|---|---|---|
| Control | G01 | 0.0 | |
| PM060184 | G02 | 16.0 | |
| | G03 | 4.0 | |
| | G04 | 1.0 | |
| Comp. **2** | G05 | 2.0 | Q7dx3, IV |
| | G06 | 0.5 | |
| | G07 | 0.125 | |
| Comp. **3** | G08 | 1.0 | |
| | G09 | 0.25 | |
| | G10 | 0.0625 | |

Treatments were initiated on Day 0.

Animals in the placebo group were sacrificed due to tumor volume (> 2,000 mm³) and/or tumor necrosis between days 9 and 14. In this experiment, HGC-27 tumors had a doubling time of 2.9 days.

Treatment with compounds 2 and 3 caused, in tumor bearing animals, a high but reversible mean body weight reduction. Compound 2 (2.0 mg/kg) treatment caused a mean body weight reduction of ca. -13.1% recorded on Day 2 and, compound **3** (1.0 mg/kg) ca. -13.1% on Day 2. When the animals were treated with PM060184 at 16 mg/kg a slight body weight decrease was recorded on Day 2 (ca. -4.1%). All the treated animals returned back to normal when the treatments stopped. No other clinical signs of systemic toxicity were seen.

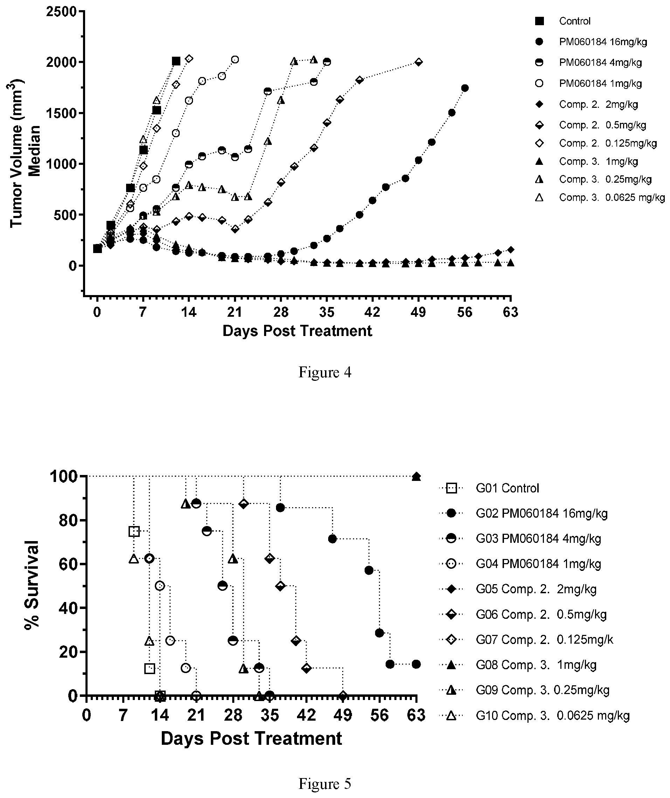

Tumor growth curves are displayed in Figure 4. All treatments showed a strong and dose-dependent antitumor activity, in HGC-27 tumor xenografts. During the experiment a 100% of animals treated with the highest dose of compound **2**(2.0 mg/kg) or compound **3** (1.0 mg/kg), were in complete tumor remission status. The mean time frame during which the animals treated with compound **2** at 2.0 mg/kg stayed with tumor remission was higher than 29.1 days and higher than 31.5 days for animals treated with compound **3** at 1.0 mg/kg; the last observed day was day 63. In addition, three out of eight animals, treated with PM060184 at 16 mg/kg (high-dose), reached a complete remission that lasted a mean of 18 days. The placebo-treated group had a median (IQR) tumor volume of 1139 (985.9-1364) and 2008 (2008-2008) mm³ on days 7 and 14, respectively. On days 7, 14, 21, 28 and 35, PM060184-treated animals, at 16.0 mg/kg, had a median (IQR) tumor volume of 250.7 (212.8-433.0), 124.8 (102.8-213.2), 88.5 (68.2-118.3), 114.4 (63.7-155.1) and 267.3 (100.4-414.0) mm³, respectively. On the same days, compound **2-**treated animals, at 2.0 mg/kg, had a median (IQR) tumor volume of 313.6 (237.3-482.4), 143.2 (96.1-223.6), 75.0 (51.6-75.0), 42.5 (37.0-63.9) and 32.9 (20.4-36.6) mm³, respectively. On days 7, 14, 21, 28 and 35, compound **3**-treated animals, at 1.0 mg/kg, had a median (IQR) tumor volume of 366.0 (300.1-479.8), 177.1 (142.3-256.0), 75.0 (65.6-84.4), 66.7 (38.2-73.7) and 27.3 (21.4-41.4) mm³, respectively. Compared to placebo, all compound-treated animals at their highest dose experienced a very strong, statistically significant tumor reduction on day 7, that being the last measurement day in the euthanized, placebo-treated group. During the treatment administration period, no differences were seen between the groups administered with PM060184 vs compounds **2** and **3** (high dose) but, once the treatments were stopped, the groups treated with **2** or **3** experienced a big difference, in terms of antitumor activity, statistically significant, p=0.0047 on day 28 and p=0.0070 on day 35, for the group treated with **2** at 2 mg/kg and p=0.0379 on day 28 and p=0.0129 on day 35, for the group treated with **3** at 1 mg/kg compared to PM060184-treated group at 16 mg/kg **(Table 10).**

**Table 10. Tumor volumes (TV) obtained in mice bearing HGC-27 xenografts and treated with PM060184, 2 and 3.**

| **Compound** | **Day** | **TV, mm3 median (IQR)** | ***P*** | ***P*** | ***P*** |
|---|---|---|---|---|---|
| G01 Placebo | 7 | 1139 (985.9-1364) | | | |
| | 14 | 2008 (2008-2008) | | | |
| G02 PM060184, 16 mg/kg | 7 | 250.7 (212.8-433.0) | 0.0002^{a} | | |
| | 14 | 124.8 (102.8-213.2) | -^{b} | | |
| | 21 | 88.5 (68.2-118.3) | -^{b} | | |
| | 28 | 114.4 (63.7-155.1) | -^{b} | | |
| | 35 | 267.3 (100.4-414.0) | -^{b} | | |
| G05 Comp. **2**, 2 mg/kg | 7 | 313.6 (237.3-482.4) | 0.0002^{a} | -^{c} | |
| | 14 | 143.2 (96.1-223.6) | -^{b} | -^{c} | |
| | 21 | 75.0 (51.6-75.0) | -^{b} | -^{c} | |
| | 28 | 42.5 (37.0-63.9) | -^{b} | 0.0047^{c} | |
| | 35 | 32.9 (20.4-36.6) | -^{b} | 0.0070^{c} | |
| G06 Comp **3,** 1mg/kg | 7 | 366.0 (300.1-479.8) | 0.0002^{a} | -^{d} | -^{e} |
| | 14 | 177.1 (142.3-256.0) | -^{b} | -^{d} | -^{e} |
| | 21 | 75.0 (65.6-84.4) | -^{b} | -^{d} | -^{e} |
| | 28 | 66.7 (38.2-73.7) | -^{b} | 0.0379^{d} | -^{e} |
| | 35 | 27.3 (21.4-41.4) | -^{b} | 0.0129^{d} | -^{e} |
| Data are presented as medians and interquartile range (IQR) | | | | | |
| ^{a} *P* value for Mann-Whitney *U* test respect to placebo treated. | | | | | |
| ^{b} No placebo group to compare with | | | | | |
| ^{c} *P* value for Mann-Whitney *U* test, comparison PM060184-treated group respect to comp. **2**-treated group. | | | | | |
| ^{d} *P* value for Mann-Whitney *U* test, comparison PM060184-treated group respect to comp. **3**-treated group. | | | | | |
| ^{e} *P* value for Mann-Whitney *U* test, comparison comp **2**-treated group respect to comp. **3**-treated group. | | | | | |

**Table 11. Survival and statistic result for mice bearing HGC-27 xenografted tumors and treated with PM060184, 2 and 3.**

| **Group** | **Treatment** | **Dose (mg/kg)** | **Median survival time (days)** | ***P*** | ***P*** | ***P*** |
|---|---|---|---|---|---|---|
| 01 | Control | 0.0 | 12 | | | |
| 02 | PM060184 | 16.0 | 56 | 0.0001^{a} | | |
| 03 | | 4.0 | 27 | <0.0001^{a} | | |
| 04 | | 1.0 | 15 | 0.0103^{a} | | |
| 05 | Comp. **2** | 2.0 | ud | 0.0002^{a} | 0.0007^{b} | |
| 06 | | 0.5 | 38.5 | <0.0001^{a} | 0.0007^{b} | |
| 07 | | 0.125 | 14 | 0.0281^{a} | -^{b} | |
| 08 | Comp. **3** | 1.0 | ud | <0.0001^{a} | 0.0007^{c} | -^{d} |
| 09 | | 0.25 | 30 | <0.0001^{a} | -^{c} | 0.0004^{d} |
| 10 | | 0.0625 | 12 | -^{a} | 0.0152^{c} | -^{d} |
| *^{a} P* value for log rank test, treated groups compared to placebo group. | | | | | | |
| ^{b} *P* value for log rank test, comparison PM060184-treated group respect to comp. **2**-treated group. | | | | | | |
| ^{c} *P* value for log rank test, comparison PM060184-treated group respect to comp. **3**-treated group. | | | | | | |
| ^{d} *P* value for log rank test, comparison comp **2**-treated group respect to comp. **3**-treated group. ud, undetermined. The group not reached mortality. | | | | | | |

Kaplan-Meier survival curves are shown in Figure 5.

PM060184 treatment statistically significantly increased the survival time compared to placebo-treated group at 16.0 mg/kg (p=0.0001), 4.0mg/kg (p<0.0001), or 1.0 mg/kg (p=0.0103).

Compound **2** treatment, statistically significantly increased the survival time compared to the placebo-treated group at 2.0 mg/kg (p=0.0002), 0.5 mg/kg (p<0.0001) and 0.125 mg/kg (p=0.0281). Comparing PM060184 and compound **2** treatments, at high and medium dose, the compound **2** treated group resulted in a statistically significant increase in the survival time compared to the PM060184-treated group (p=0.0007) and when compounds **2** and **3** treatments were compared, at medium dose, compound **2** treated group resulted in a statistically significant (p=0.0004) increase in the survival time compared to compound 3-treated group. For clarity the high dose lines for compounds **2** and **3** overlay.

Compound **3** treatment, statistically significantly increased the survival time compared to the placebo-treated group at 1.0 mg/kg (p<0.0001) or 0.25 mg/kg (p<0.0001). Comparing PM060184 and compound **3** treatments, both at high dose, the compound **3** treated group resulted in a statistically significant (p=0.0007) increase in the survival time compared to PM060184-treated group.

### Example 7. In vivo studies to determine the effect of the compounds 2 and 3 in a colon xenograft model, HCT-116

The experiment was carried out as previously explained. When tumors reached ca. 195-205 mm³, tumor bearing animals (N =10/group) were randomly allocated into the following treatment groups:

| **Group** | **Group number** | **Dose (mg/kg)** | **Schedule** |
|---|---|---|---|
| Control | G01 | 0.0 | |
| PM060184 | G02 | 16.0 | |
| | G03 | 4.0 | |
| | G04 | 1.0 | |
| Comp. **2** | G05 | 2.0 | Q7dx3, IV |
| | G06 | 0.5 | |
| | G07 | 0.125 | |
| Comp. **3** | G08 | 1.0 | |
| | G09 | 0.25 | |
| | G10 | 0.0625 | |

Treatments were initiated on Day 0.

Animals in the placebo group were sacrificed due to tumor volume (> 2,000 mm³) and/or tumor necrosis between days 12 and 19. In this experiment, HCT-116 tumors had a doubling time of 3.9 days.

Treatment with compounds **2** and **3** caused, in tumor bearing animals, a high but reversible mean body weight reduction. Compound **2** (2.0 mg/kg) treatment caused a mean body weight reduction of (ca. -13.5%) recorded on Day 9 and compound **3** (1.0 mg/kg) (ca. -16.2%) recorded on Day 9. When the animals were treated with PM060184 at 16 mg/kg a slight body weight decrease was recorded on Day 2 (ca. -3.2%). All the treated animals returned to normal values when the treatments were finalized. No other clinical signs of systemic toxicity were seen.

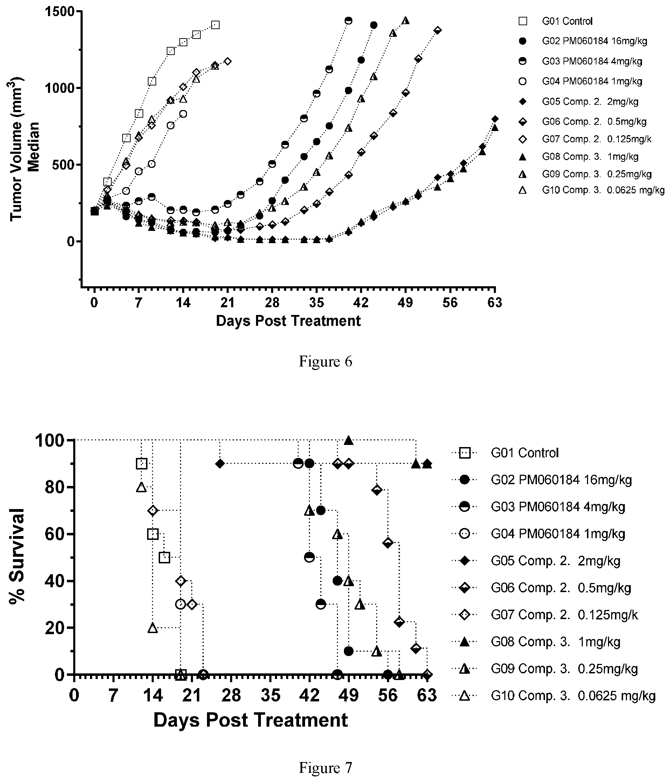

Tumor growth curves are displayed in Figure 6. All treatments showed a strong and dose-dependent antitumor activity, in HGC-27 tumor xenografts. During the experiment, a 100% of animals treated with the highest dose of compound **2** (2.0 mg/kg) or compound **3** (1.0 mg/kg), were in complete tumor remission. The mean time frame during which the animals treated with compound **2** at 2.0 mg/kg stayed with tumor remission was 25.8 days and 23.7 days for animals treated with compound **3** at 1.0 mg/kg. Moreover, 6/10 animals, treated with PM060184 at 16 mg/kg (high-dose), 4/10 animals, treated with compound **2** at 0.5 mg/kg (medium-dose) and 1/10 animals, treated with compound **3** at 0.25 mg/kg (medium-dose), reached a complete remission that lasted a mean of 5.8 days, 9 days and 2 days, respectively. Placebo-treated group had a median (IQR) tumor volume of 836.3 (737.2-911.9), 1299 (1141-1420) mm³ on days 7 and 14, respectively. On days 7, 14, 21, 28 and 35, PM060184-treated animals, at 16.0 mg/kg, had a median (IQR) tumor volume of 140.5 (123.0-157.3), 59.2 (50.7-84.2), 63.8 (43.6-80.0), 265.7 (195.2-284.3) and 650.2 (516.7-732.6) mm³, respectively. On the same days, compound 2-treated animals, at 2.0 mg/kg, had a median (IQR) tumor volume of 135.5 (114.1-154.5), 57.2 (47.1-62.9), 25.0 (13.5-34.0), 13.5 (13.5-32.0) and 13.5 (13.5-13.5) mm³, respectively. On days 7, 14, 21, 28 and 35, compound 3-treated animals, at 1.0 mg/kg, had a median (IQR) tumor volume of 121.6 (86.3-164.8), 55.6 (16.2-82.3), 32.0 (4.0-40.0), 13.5 (4.0-13.5) and 13.5 (13.5-13.5) mm³, respectively. Compared to placebo, all compound-treated animals at their highest dose experienced a very strong, statistically significant tumor reduction from day 7 to day 14, that being the last measurement day in the euthanized, placebo-treated group. During the treatment administration period, no differences were seen between the groups administered with PM060184 vs compounds **2** and **3** (high dose) but, once the treatments were finalized, the groups treated with **2** or **3** experienced a big difference, in terms of antitumor activity, statistically significant, p=0.0002 from day 21 to day 35 for the group treated with compound **2** at 2 mg/kg and p=0.0005 on day 21, p=0.0002 on day 28 and p<0.0001 on day 35 for the group treated with compound **3** at 1 mg/kg, compared to PM060184-treated group at 16 mg/kg (**Table 12**).

**Table 12. Tumor volumes (TV) obtained in mice bearing HCT-116 xenografts and treated with PM060184, 2 and 3.**

| **Compound** | **Day** | **TV, mm3 median (IQR)** | ***P*** | ***P*** | ***P*** |
|---|---|---|---|---|---|
| G01 Placebo | 7 | 836.3 (737.2-911.9) | | | |
| | 14 | 1299 (1141-1420) | | | |
| G02 PM060184, 16 mg/kg | 7 | 140.5 (123.0-157.3) | <0.0001^{a} | | |
| | 14 | 59.2 (50.7-84.2) | <0.0001^{a} | | |
| | 21 | 63.8 (43.6-80.0) | -^{b} | | |
| | 28 | 265.7 (195.2-284.3) | -^{b} | | |
| | 35 | 650.2 (516.7-732.6) | -^{b} | | |
| G05 Comp. **2**, 2 mg/kg | 7 | 135.5 (114.1-154.5) | <0.0001^{a} | -^{c} | |
| | 14 | 57.2 (47.1-62.9) | <0.0001^{a} | -^{c} | |
| | 21 | 25.0 (13.5-34.0) | -^{b} | 0.0002^{c} | |
| | 28 | 13.5 (13.5-32.0) | -^{b} | 0.0002^{c} | |
| | 35 | 13.5 (13.5-13.5) | -^{b} | 0.0002^{c} | |
| G06 Comp **3**, 1mg/kg | 7 | 121.6 (86.3-164.8) | <0.0001^{a} | -^{d} | -^{e} |
| | 14 | 55.6 (16.2-82.3) | 0.0003^{a} | -^{d} | -^{e} |
| | 21 | 32.0 (4.0-40.0) | -^{b} | 0.0005^{d} | -^{e} |
| | 28 | 13.5 (4.0-13.5) | -^{b} | 0.0002^{d} | 0.0444^{e} |
| | 35 | 13.5 (13.5-13.5) | -^{b} | <0.0001^{d} | -^{e} |
| Data are presented as medians and interquartile range (IQR) | | | | | |
| *^{a} P* value for Mann-Whitney *U* test respect to placebo treated. | | | | | |
| ^{b} No placebo group to compare with | | | | | |
| ^{c} *P* value for Mann-Whitney *U* test, comparison PM060184-treated group respect to comp. **2**-treated group. | | | | | |
| ^{d} *P* value for Mann-Whitney *U* test, comparison PM060184-treated group respect to comp. **3**-treated group. | | | | | |
| ^{e} *P* value for Mann-Whitney *U* test, comparison comp **2**-treated group respect to comp. **3**-treated group. | | | | | |

**Table 13. Survival and statistic result for mice bearing HCT-116 xenografted tumors and treated with PM060184, 2 and 3.**

| **Group** | **Treatment** | **Dose (mg/kg)** | **Median survival time (days)** | ***P*** | ***P*** | ***P*** |
|---|---|---|---|---|---|---|
| 01 | Control | 0.0 | 17.5 | | | |
| 02 | PM060184 | 16.0 | 47 | 0.0001^{a} | | |
| 03 | | 4.0 | 43 | <0.0001^{a} | | |
| 04 | | 1.0 | 19 | 0.0047^{a} | | |
| 05 | Comp. **2** | 2.0 | ud | <0.0001^{a} | 0.0012^{b} | |
| 06 | | 0.5 | 58 | <0.0001^{a} | <0.0001^{b} | |
| 07 | | 0.125 | 19 | -^{a} | -^{b} | |
| 08 | Comp. **3** | 1.0 | ud | <0.0001^{a} | <0.0001^{c} | -^{d} |
| 09 | | 0.25 | 49 | <0.0001^{a} | 0.0076^{c} | 0.0033^{d} |
| 10 | | 0.0625 | 14 | -^{a} | 0.0004^{c} | 0.0070^{d} |
| *^{a} P* value for log rank test, treated groups compared to placebo group. | | | | | | |
| ^{b} *P* value for log rank test, comparison PM060184-treated group respect to comp. **2**-treated group. | | | | | | |
| ^{c} *P* value for log rank test, comparison PM060184-treated group respect to comp. **3**-treated group. | | | | | | |
| ^{d} *P* value for log rank test, comparison comp **2**-treated group respect to comp. **3**-treated group. ud, undetermined. The group not reached mortality. | | | | | | |

Kaplan-Meier survival curves are shown in Figure 7.

PM060184 treatment, statistically significantly increased the survival time compared to placebo-treated group at 16.0 mg/kg (p<0.0001), 4.0mg/kg (p<0.0001) or 1.0 mg/kg (p=0.0047). Placebo-treated median survival time was 17.5 vs. 47, 43 or 19 days for PM060184-treated groups (high, medium or low dose, respectively).

Compound **2** treatment, statistically significantly increased the survival time compared to placebo-treated group at 2.0 mg/kg (p<0.0001), 0.5 mg/kg (p<0.0001), but not at 0.125 mg/kg. Comparing PM060184 and compound **2** treatments, both at high and medium dose, compound **2** treated group resulted in a statistically significant increase in the survival time compared to the PM060184-treated group (p= 0.0012 or p<0.0001, respectively) and when compounds **2** and **3** treatments were compared, at medium and low dose, the compound **2** treated group resulted in a statistically significant increase in the survival time compared to compound **3**-treated group, p=0.0033 and p=0.0070, respectively.

Compound **3** treatment statistically significantly increased the survival time compared to placebo-treated group at 1.0 mg/kg (p<0.0001), 0.25 mg/kg (p<0.0001) but not at 0.125 mg/kg. When compound **3** and PM060184 treatments were compared, at high and medium dose, compound **3** treated group resulted in a statistically significant increase in the survival time compared to the PM060184-treated group, p<0.0001 and p=0.0076, respectively.

In conclusion, it can be seen that compounds **2** and **3** both have surprisingly improved activity versus prior art compounds PM060184 and PM050489.

*In vitro,* the compound **2** is between 3.8 to 8.2 times more active than PM060184; and the compound **3** is between 1.3 to 3.7 times more active than PM060184. This is seen across A549, HT29, MDA-MB-231 and PSN-1 cell lines. This is seen from Example 3.

*In vivo*, the compounds **2** and **3** show a remarkable improvement in efficacy over PM060184 across a wide range of cancer xenograft models. In H460, HGC27 and HCT116 xenograft models (which model non-small cell lung cancer, gastric cancer and colorectal carcinoma), compounds **2** and **3** both show stastitically significant improvements not only in antitumor activity but also in survival time across all three cell lines. See Examples 5 to 7.

This is also shown for PM050489 in the *in vitro* bioassays in Example 3 where it can be seen that **2** and **3** both have improved *in vitro* activity versus prior art compound PM050489. Compound 2 is between 1.9 to 7.9 times more active than reference compound PM050489. Compound 3 is between 1.3 to 1.8 times more active than reference compound PM050489. This is seen across A549, HT29, MDA-MB-231 and PSN-1 cell lines. This is also shown in the *in vivo* xenograft models, where there is a significant improvement in efficacy over PM050489 in the H460 xenograft model. This is seen from Example 3 for *in vitro* data and from the Comparative Example and Example 5 for *in vivo* data.

### References:

1. WO 2007/144423
2. Mar. Drugs 2019, 17, 329; doi:10.3390/md17060329
3. Martínez-Díez et al, PM060184, a new tubulin binding agent with potent antitumor activity including P-glycoprotein over-expressing tumors. Biochem. Pharmacol. 2014, 88, 291-302.
4. Pera, B. et al, New interfacial microtubule inhibitors of marine origin, PM050489/PM060184, with potent antitumor activity and a distinct mechanism. ACS Chem. Biol. 2013, 8, 2084-2094.
5. Martin, MJ. et al. Isolation and first total synthesis of PM050489 and PM060184, two new marine anticancer compounds. J. Am. Chem. Soc. 2013, 135, 10164-10171.
6. Prota, A.E. et al. A new tubulin-binding site and pharmacophore for microtubule-destabilizing anticancer drugs. Proc. Natl. Acad. Sci. USA 2014, 111, 13817-13821
7. Galmarini, C.M. et al. Plocabulin, a novel tubulin-binding agent, inhibits angiogenesis by modulation of microtubule dynamics in endothelial cells. BMC Cancer 2018, 18, 164
8. Pantazopoulou, A. et al. Molecular basis of resistance to the microtubule-depolymerizing antitumor compound plocabulin. Sci. Rep. 2018, 8:8616
9. Mar. Drugs 2019, 17, 648, doi: 10.3390/md17110648
10. Elez, E. et al. First-in-human phase I study of the microtubule inhibitor plocabulin in patients with advanced solid tumors. Invest. New Drugs, 2019, 37, 674-683
11. Navarrete, K.R. et al. Interdimeric curvature in tubulin-tubulin complexes delineates the microtubule-destabilizing properties of plocabulin. J. Chem. Inf. Model. 2020, 60, 4076-4084
12. Wang, Y. et al. Plocabulin, a novel tubulin inhibitor, has potent antitumor activity in patient-derived xenograft models of gastrointestinal stromal tumors. Trans. Oncol. 2020, 13(11), 100832
13. Skehan P, Storeng R, Scudiero D, Monks A, McMahon J, Vistica D, Warren JT, Bokesch H, Kenney S, Boyd MR. New colorimetric cytotoxicity assay for anticancer-drug screening. J Natl Cancer Inst. 1990 Jul 4;82(13):1107-12. doi: 10.1093/jnci/82.13.1107. PMID: 2359136.
14. Vichai V, Kirtikara K. Sulforhodamine B colorimetric assay for cytotoxicity screening. Nat Protoc. 2006;1(3):1112-6. doi: 10.1038/nprot.2006.179. PMID: 17406391.

## Claims

1. A compound of formula I: 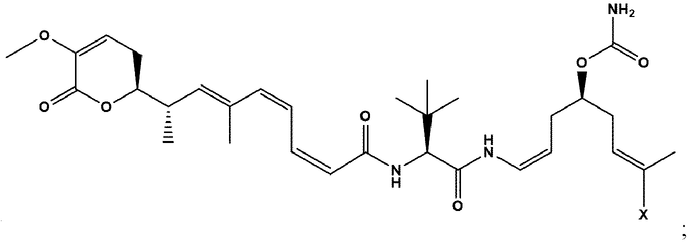 wherein X = F or Me.

2. The compound according to claim 1 of formula II:

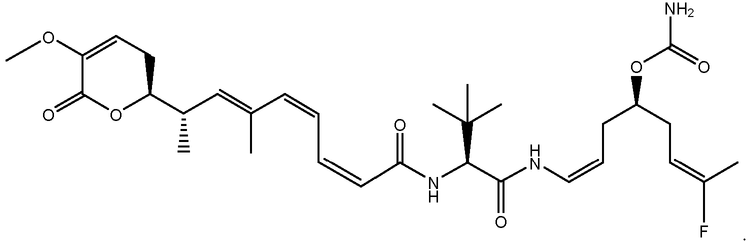

3. The compound according to claim 1 of formula III:

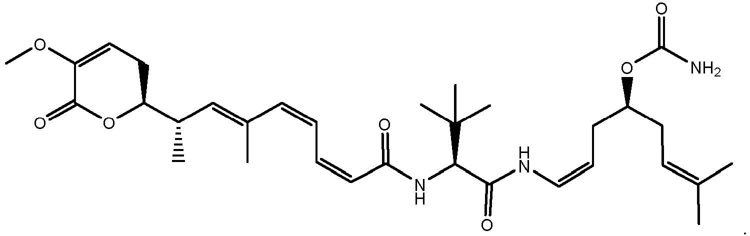

4. A pharmaceutical composition comprising a compound according to any one of claims 1 to 3 and a pharmaceutically acceptable carrier.

5. A dosage form comprising a pharmaceutical composition according to claim 4.

6. A compound according to any one of claims 1 to 3, or a pharmaceutical composition according to claim 4, or a dosage form according to claim 5, for use as a medicament.

7. A compound according to any one of claims 1 to 3, or a pharmaceutical composition according to claim 4, or a dosage form according to claim 5, for use in the treatment of cancer.

8. The compound, pharmaceutical composition or dosage form for use according to claim 7, wherein the cancer is a solid tumor, optionally selected from lung cancer, non-small cell lung cancer, colorectal cancer, breast cancer, pancreatic cancer and gastric cancer.

9. Use of a compound according to any one of claims 1 to 3, or a pharmaceutical composition according to claim 4, or a dosage form according to claim 5, in the manufacture of a medicament for the treatment of cancer; optionally wherein the cancer is a solid tumor; including a cancer selected from lung cancer, non-small cell lung cancer, colorectal cancer, breast cancer, pancreatic cancer and gastric cancer.

10. An in vitro method of inhibiting cancer cell growth, comprising contacting cancer cells with a compound according to any one of claims 1 to 3, or a pharmaceutical composition according to claim 4, or a dosage form according to claim 5; optionally wherein the cancer cell is a solid tumor; including a cancer selected from lung cancer, non-small cell lung cancer, colorectal cancer, breast cancer, pancreatic cancer and gastric cancer.

11. A kit comprising a therapeutically effective amount of a compound according to any one of claims 1 to 3 and a pharmaceutically acceptable carrier, or a pharmaceutical composition according to claim 4, or a dosage form according to claim 5.

12. The kit according to claim 11 further comprising instructions for use of the compound in the treatment of cancer, and more preferably a solid tumor, yet more preferably a cancer selected from lung cancer, non-small cell lung cancer, colorectal cancer, breast cancer, pancreatic cancer and gastric cancer.

13. A process for the manufacture of a compound of formula I, comprising reacting a compound of formula V with a suitable reagent for the synthesis of carbamates to give a compound of formula I 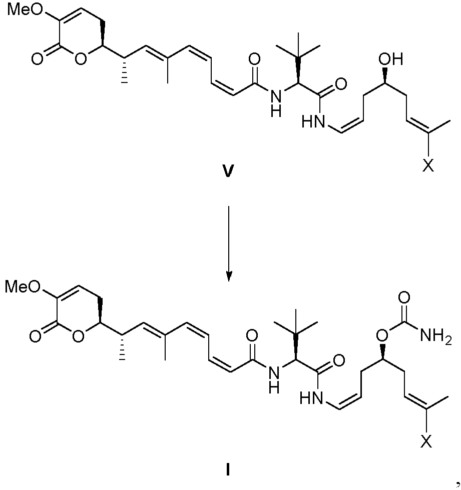 wherein X = F or Me.

14. An intermediate compound of formula **V**, **VII**, **IX** or **XI**, useful for the manufacture of a compound of formula I as defined in claim 1 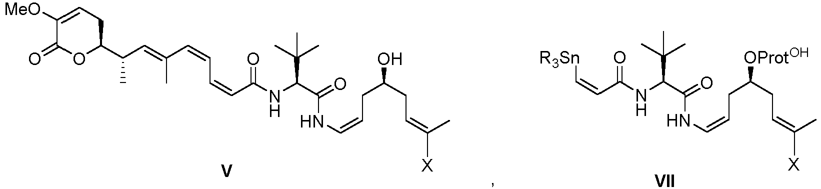 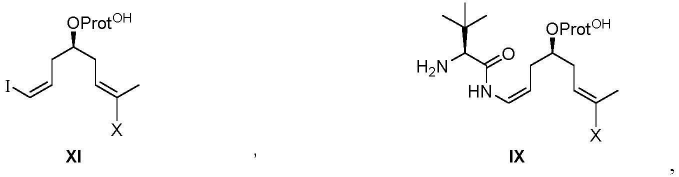 wherein R₃ is an unsubstituted C₁-C₄ alkyl group, ProtOH is a protecting group for hydroxy and X is as defined for formula **I**.

15. The intermediate compound according to claim 14 of formula **V** 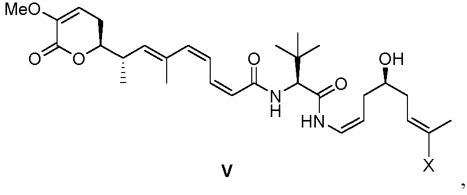 wherein X = F or Me.

## Patentansprüche

1. Verbindung der Formel I: 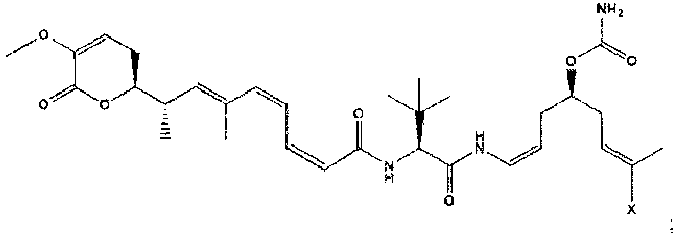 wobei X = F oder Me ist.

2. Verbindung gemäß Anspruch 1 der Formel II:

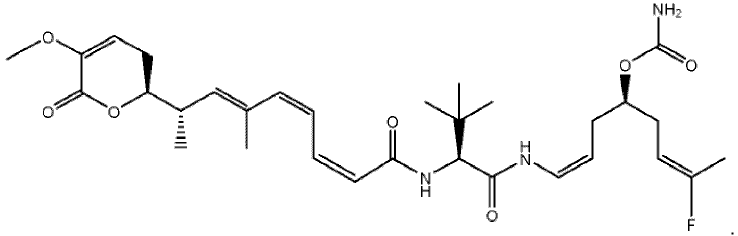

3. Verbindung gemäß Anspruch 1 der Formel III:

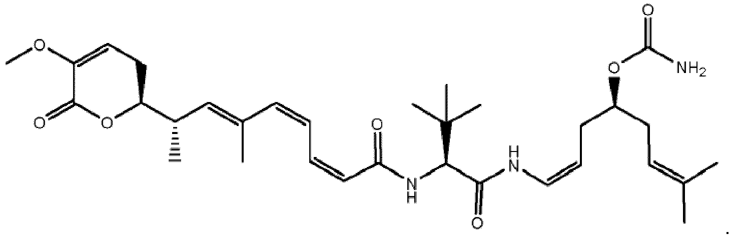

4. Pharmazeutische Zusammensetzung, umfassend eine Verbindung gemäß einem der Ansprüche 1 bis 3 und einen pharmazeutisch annehmbaren Träger.

5. Darreichungsform, umfassend eine pharmazeutische Zusammensetzung gemäß Anspruch 4.

6. Verbindung gemäß einem der Ansprüche 1 bis 3 oder pharmazeutische Zusammensetzung gemäß Anspruch 4 oder Darreichungsform gemäß Anspruch 5 zur Verwendung als Medikament.

7. Verbindung gemäß einem der Ansprüche 1 bis 3 oder pharmazeutische Zusammensetzung gemäß Anspruch 4 oder Darreichungsform gemäß Anspruch 5 zur Verwendung bei der Behandlung von Krebs.

8. Verbindung, pharmazeutische Zusammensetzung oder Darreichungsform zur Verwendung gemäß Anspruch 7, wobei der Krebs ein solider Tumor ist, der gegebenenfalls aus Lungenkrebs, nichtkleinzelligem Lungenkrebs, Darmkrebs, Brustkrebs, Pankreaskrebs und Magenkrebs ausgewählt ist.

9. Verwendung einer Verbindung gemäß einem der Ansprüche 1 bis 3 oder einer pharmazeutischen Zusammensetzung gemäß Anspruch 4 oder einer Darreichungsform gemäß Anspruch 5 bei der Herstellung eines Medikaments zur Behandlung von Krebs; wobei gegebenenfalls der Krebs ein solider Tumor ist; einschließlich einer Krebserkrankung, die aus Lungenkrebs, nichtkleinzelligem Lungenkrebs, Darmkrebs, Brustkrebs, Pankreaskrebs und Magenkrebs ausgewählt ist.

10. In-vitro-Verfahren zur Hemmung des Wachstums von Krebszellen, umfassend das In-Kontakt-Bringen von Krebszellen mit einer Verbindung gemäß einem der Ansprüche 1 bis 3 oder einer pharmazeutischen Zusammensetzung gemäß Anspruch 4 oder einer Darreichungsform gemäß Anspruch 5; wobei es sich gegebenenfalls bei der Krebszelle um einen soliden Tumor handelt; einschließlich einer Krebserkrankung, die aus Lungenkrebs, nichtkleinzelligem Lungenkrebs, Darmkrebs, Brustkrebs, Pankreaskrebs und Magenkrebs ausgewählt ist.

11. Kit, umfassend eine theraapeutisch wirksame Menge einer Verbindung gemäß einem der Ansprüche 1 bis 3 und eines pharmazeutisch annehmbaren Trägers oder einer pharmazeutischen Zusammensetzung gemäß Anspruch 4 oder einer Darreichungsform gemäß Anspruch 5.

12. Kit gemäß Anspruch 11, weiterhin umfassend Anweisungen zur Verwendung der Verbindung bei der Behandlung von Krebs und besonders bevorzugt eines soliden Tumors, ganz besonders bevorzugt einer Krebserkrankung, die aus Lungenkrebs, nichtkleinzelligem Lungenkrebs, Darmkrebs, Brustkrebs, Pankreaskrebs und Magenkrebs ausgewählt ist.

13. Verfahren zur Herstellung einer Verbindung der Formel I, umfassend das Umsetzen einer Verbindung der Formel V mit einem geeigneten Reagens für die Synthese von Carbamaten, was eine Verbindung der Formel I ergibt: 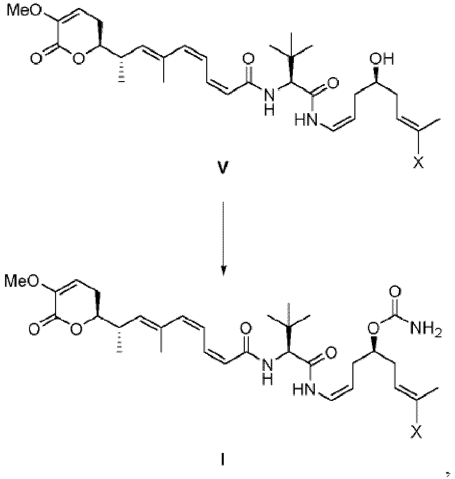 wobei X = F oder Me ist.

14. Zwischenverbindung der Formel V, VII, IX oder XI, die für die Herstellung einer Verbindung der Formel I, wie sie in Anspruch 1 definiert ist, geeignet ist: 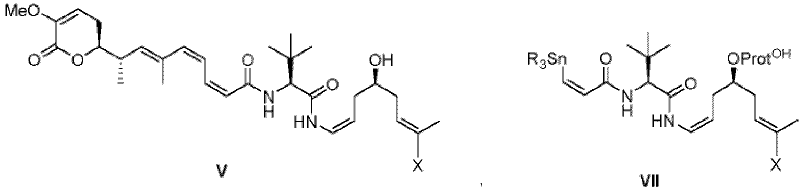 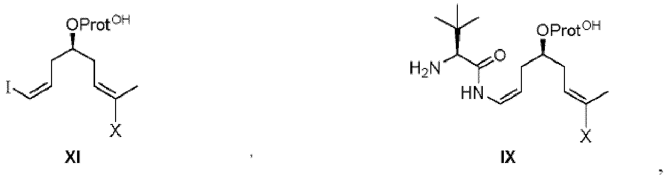 wobei R₃ eine unssubstituierte C₁-C₄-Alkylgruppe ist, ProtOH eine Schutzgruppe
für Hydroxy ist und X wie für Formel I definiert ist.

15. Zwischenverbindung gemäß Anspruch 14 der Formel V: 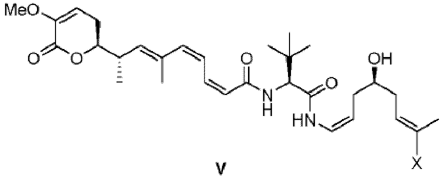 , wobei X = F oder Me ist.

## Revendications

1. Composé de formule I : 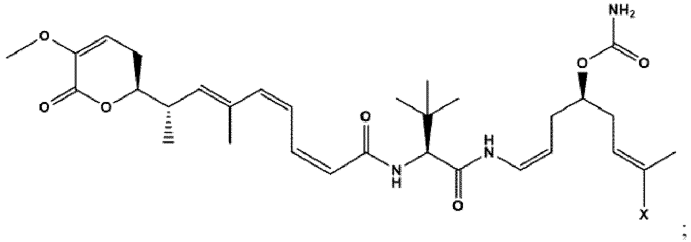 dans lequel X = F ou Me.

2. Composé selon la revendication 1 de formule II:

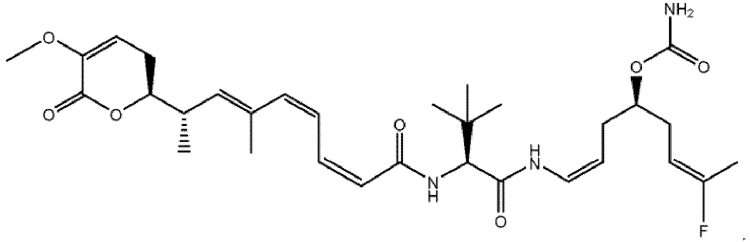

3. Composé selon la revendication 1 de formule III:

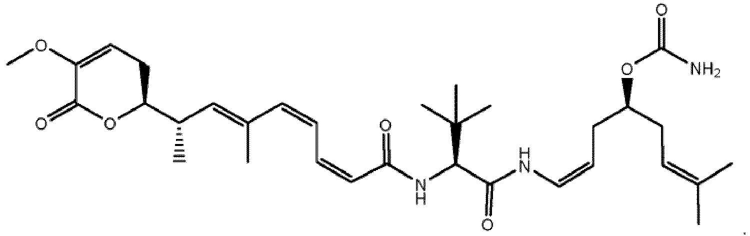

4. Composition pharmaceutique comprenant un composé selon l'une quelconque des revendications 1 à 3 et un support pharmaceutiquement acceptable.

5. Forme posologique comprenant une composition pharmaceutique selon la revendication 4.

6. Composé selon l'une quelconque des revendications 1 à 3, ou composition pharmaceutique selon la revendication 4, ou forme posologique selon la revendication 5, pour utilisation en tant que médicament.

7. Composé selon l'une quelconque des revendications 1 à 3, ou composition pharmaceutique selon la revendication 4, ou une forme posologique selon la revendication 5, pour son utilisation dans le traitement du cancer.

8. Le composé, la composition pharmaceutique ou la forme posologique pour une utilisation selon la revendication 7, dans laquelle le cancer est une tumeur solide, éventuellement choisie parmi le cancer du poumon, le cancer du poumon non à petites cellules, le cancer colorectal, le cancer du sein, le cancer du pancréas et le cancer gastrique.

9. Utilisation d'un composé selon l'une quelconque des revendications 1 à 3, ou d'une composition pharmaceutique selon la revendication 4, ou d'une forme posologique selon la revendication 5, dans la fabrication d'un médicament pour le traitement du cancer ; éventuellement dans laquelle le cancer est une tumeur solide ; y compris un cancer choisi parmi le cancer du poumon, le cancer du poumon non à petites cellules, le cancer colorectal, le cancer du sein, le cancer du pancréas et le cancer gastrique.

10. Méthode in vitro d'inhibition de la croissance des cellules cancéreuses, comprenant la mise en contact de cellules cancéreuses avec un composé selon l'une quelconque des revendications 1 à 3, ou une composition pharmaceutique selon la revendication 4, ou une forme posologique selon la revendication 5 ; éventuellement dans laquelle la cellule cancéreuse est une tumeur solide, y compris un cancer choisi parmi le cancer du poumon, le cancer du poumon non à petites cellules, le cancer colorectal, le cancer du sein, le cancer du pancréas et le cancer gastrique.

11. Kit comprenant une quantité thérapeutiquement efficace d'un composé selon l'une quelconque des revendications 1 à 3 et un support pharmaceutiquement acceptable, ou une composition pharmaceutique selon la revendication 4, ou une forme posologique selon la revendication 5.

12. Le kit selon la revendication 11 comprenant en outre des instructions pour l'utilisation du composé dans le traitement du cancer, et plus préférentiellement d'une tumeur solide, encore plus préférentiellement d'un cancer choisi parmi le cancer du poumon, le cancer du poumon non à petites cellules, le cancer colorectal, le cancer du sein, le cancer du pancréas et le cancer gastrique.

13. Procédé de fabrication d'un composé de formule I, comprenant la réaction d'un composé de formule V avec un réactif adapté pour la synthèse de carbamates pour donner un composé de formule I 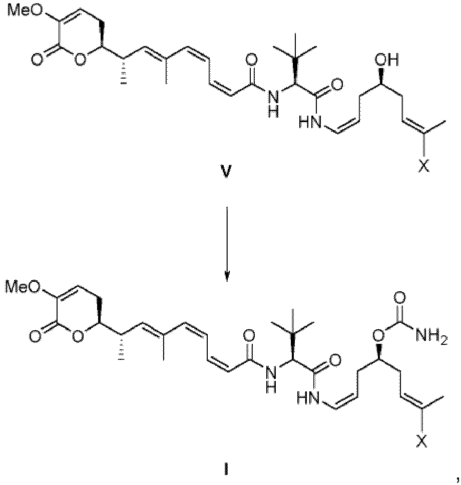 dans lequel X = F ou Me.

14. Composé intermédiaire de formule V, VII, IX ou XI, utile pour la fabrication d'un composé de formule I tel que défini dans la revendication 1 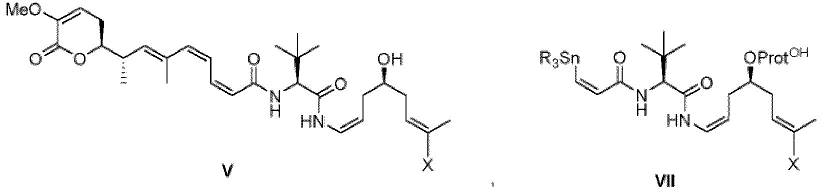 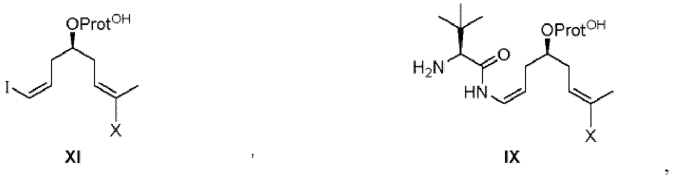 dans lequel R₃ est un groupe alkyle C₁-C₄ non substitué, ProtOH est un groupe de protection pour l'hydroxy et X est tel que défini pour la formule 1.

15. Composé intermédiaire selon la revendication 14 de formule V 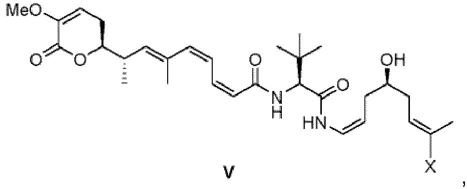 dans lequel X = F ou Me.
